# EUROPEAN PATENT SPECIFICATION

(11) **EP 2 663 614 B1**
(45) Date of publication and mention of the grant of the patent: **07.08.2019**
(21) Application number: 11849756.9
(22) Date of filing: 12.12.2011
(51) Int. Cl.: C05F 11/02, C05F 17/00, C12M 1/00

(54) **INTEGRATED COAL TO LIQUIDS PROCESS AND SYSTEM WITH CO2 MITIGATION USING ALGAL BIOMASS**
INTEGRIERTE KOHLE ZUR FLÜSSIGKEITSVERARBEITUNG UND SYSTEM MIT CO2-ABSCHWÄCHUNG UNTER VERWENDUNG EINER ALGENBIOMASSE
PROCÉDÉ ET SYSTÈME INTÉGRÉS DE TRANSFORMATION DU CHARBON EN LIQUIDES, CARACTÉRISÉS PAR UNE ATTÉNUATION DES IMPACTS DU CO2 ET FAISANT APPEL À UNE BIOMASSE ALGALE

(30) Priority: 13.12.2010 US 422645 P
(43) Date of publication of application: 20.11.2013
(60) Divisional application: 18179775.4
(73) Proprietor: Accelergy Corporation, Houston, TX 77007 (US)
(72) Inventor: FIATO, Rocco, A., Basking Ridge, NJ 07920 (US); BAUMAN, Richard, F., Billingham, WA 98229 (US); ZACZEPINSKI, Sioma, Morristown, NJ 07960 (US); BISIO, Attilio, Westfield, NJ 07091 (US)
(74) Representative: Lucas, Brian Ronald
(86) International application number: PCT/US2011/064431
(87) International publication number: WO 2012/082627

(56) References cited:
- WO-A1-2010/138620
- US-A- 4 609 456
- US-A1- 2008 236 227
- US-A1- 2008 248 953
- US-A1- 2009 286 889
- US-A1- 2010 083 575
- US-A1- 2010 112 649
- US-A1- 2010 120 104
- US-A1- 2010 159 539
- US-A1- 2010 170 144
- US-A1- 2010 255 458
- MOHAMED ELANWAR H OSMAN ET AL: "Effect of two species of cyanobacteria as biofertilizers on some metabolic activities, growth, and yield of pea plant", BIOLOGY AND FERTILITY OF SOILS ; COOPERATING JOURNAL OF INTERNATIONAL SOCIETY OF SOIL SCIENCE, SPRINGER, BERLIN, DE, vol. 46, no. 8, 12 August 2010 (2010-08-12), pages 861-875, XP019845976, ISSN: 1432-0789

## Description

### FIELD OF THE INVENTION

The present invention relates to integrated coal to liquids or electrical power, and particularly to an integrated coal or coal and biomass to liquids or electrical power processes and systems in which CO₂ emissions are substantially reduced by using CO₂ to produce algal biomass including cyanobacteria, and preferably including other photosynthetic microorganisms and the use thereof as a biofertilizer and optionally for producing synthesis gas and H₂.

### BACKGROUND OF THE INVENTION

Increases in the cost of petroleum and concerns about future shortages has led to increased interest in other carbonaceous energy resources, such as coal, tar sands, shale and the mixtures thereof. Coal is the most important of these alternative resources for reasons including the fact that vast, easily accessible coal deposits exist in several parts of the world, and the other resources contain a much higher proportion of mineral matter and a lower carbon content. Various processes have been proposed for converting such materials to liquid and gaseous fuel products including gasoline, diesel fuel, aviation fuel and heating oils, and, in some cases, to other products such as lubricants and chemicals.

A number of problems have hampered widespread use of coal and other solid fossil energy sources that include the relatively low thermal efficiency of indirect coal-to-liquids (CTL) conversion methods, such as Fischer Tropsch (FT) synthesis and methanol-to-liquids (MTL) conversion. The conversion of coal, which has a H/C ratio of approximately 1:1, to hydrocarbon products, such as fuels that have H/C ratio of something greater than 2:1 results in at least half of the carbon in the coal being converted to CO₂, and thereby wasted. Additionally, the fact that, heretofore, a large amount of greenhouse gas (GHG), particularly in the form of CO₂, is emitted as a waste product in the conversion of coal to useful products has caused CTL processes to be disfavored by many from an environmental point of view. It has been proposed to at least partially overcome the GHG problem by capturing and sequestering the carbon dioxide by reinjecting it into subterranean formations. Such an arrangement has the disadvantages of being expensive, of further reducing the process energy efficiency, of requiring the availability of appropriate subterranean formations somewhere in the vicinity of the conversion facility, of concerns about the subsequent escape into the atmosphere of the carbon dioxide, and of the waste of the energy potential of the carbon content of the carbon dioxide.

Direct coal liquefaction (DCL) methods have been developed for liquefying carbonaceous materials such as coal that have advantages in many applications to conversion by FT synthesis, including substantially higher thermal efficiency and lower CO₂ emissions. Such direct liquefaction methods typically involve heating the carbonaceous material in the presence of a donor solvent, and optionally a catalyst, in a hydrogen containing atmosphere to a temperature in the range of about 700° to 850°F to break down the coal structure into free radicals that are quenched to produce liquid products. The catalyst can typically be very finely divided iron or molybdenum or mixtures thereof. The molybdenum catalyst can be prepared in situ from a phosphomolybdic acid (PMA) precursor. Hybrid coal liquefaction systems involving both direct liquefaction and FT synthesis, or direct liquefaction and biomass conversion have been proposed in which the FT synthesis or biomass conversion provides additional hydrogen for the direct liquefaction, thereby reducing carbon dioxide emissions. Hybrid coal liquefaction systems involving all three of direct liquefaction, FT synthesis, and biomass conversion have also been proposed. None of these proposed arrangements, however, achieve the combination of thermal efficiency, low cost and substantially reduced GHG emissions that would be required for them to be economically and environmentally attractive. There remains an important need for economical coal and biomass to liquids conversion processes with reduced carbon dioxide emissions and efficient use of carbon resources.

Coal fired power plants generate about half of the United States' electricity and are expected to continue supplying a large portion of the nation's electricity in the future. According to the Department of Energy's (DOE) Energy Information Administration (EIA), coal will provide 44 percent of the electricity in 2035 in the United States. The critical role that coal plays in supplying electricity is due in part to the large coal reserves in the United States, which some estimate will last about 240 years at current consumption levels, and the relatively low cost of this energy supply. However, coal power plants also currently account for about one-third of the nation's emissions of CO₂, the most prevalent GHG. In the United States and elsewhere, these concerns have increased focus on developing and using technologies to limit CO₂ emissions from coal power plants while allowing coal to remain a viable source of energy.

It has been proposed to use CO₂ emissions produced by coal conversion facilities to make algae and oxygen. Lipids in the algae can then be converted directly to liquid fuels, and the residual biomass, or if desired, the entire algae can be processed in indirect conversion processes such as Fischer Tropsch, to produce hydrogen and liquid fuels. US 2009/286889 (Fiato) discloses an integrated process for converting a coal containing feed to liquids comprising the steps of: a. converting a major portion of the coal containing feed to liquids by direct liquefaction; b. gasifying a minor portion of the coal containing feedstock to produce syngas; c. gasifying biomass to produce syngas; d. converting syngas produced by steps b and c to methanol; e. reacting CO2 produced by step b with ammonia to form urea; f. reacting urea produced by step e with methanol produced by step c to produce dimethyl carbonate and ammonia; and g. using ammonia produced in step a and/or e as a reactant in step f.

US 2010/159539 (Ascon) discloses methods and systems for producing biofuel and bioenergy products using, as starting raw material, xenobiotic materials or compounds which may originate from industrial or chemical plants, municipal waste, pharmaceutical products, cosmetic and personal care products, or other sources, and may include aliphatic and aromatic hydrocarbons, chlorinated organic solvents and other halogenated hydrocarbons, as well as heteroaromatic compounds. These materials act as a carbon source to support the metabolism of xenobiotic-degrading microorganisms, thereby producing biomass and/or biogas that may be converted to bioenergy products by microbial synthesis. For example, the biomass may be converted to products such as hydrogen gas, ethanol, methanol, butanol, methane and biodiesel. The microbial breakdown (decomposition) of xenobiotic materials is coupled with the microbial synthesis of biofuel, thereby supplying needed (inexpensive) energy products, while reducing environmental pollution and contamination, and reducing the costs associated with disposal of hazardous waste.

### SUMMARY OF THE INVENTION

The present invention provides a method for converting a solid carbonaceous material to liquid fuels and cyanobacteria based biofertilizer, comprising the steps of: (a) directly liquefying a coal containing solid carbonaceous material by subjecting said material to elevated temperatures and pressures in the presence of a solvent and a molybdenum containing catalyst for a time sufficient for producing hydrocarbon liquids and byproduct CO₂; (b) upgrading hydrocarbon liquids produced by step (a) to liquid fuels and byproduct ammonia; (c) producing hydrogen and byproduct CO₂ from a carbonaceous feed, and supplying at least a portion of said hydrogen as inputs to said direct liquefaction and said upgrading steps; (d) reproducing nitrogen-fixing cyanobacteria in a photobioreactor with the use of byproduct C0₂ produced by one or both of said direct liquefaction and hydrogen producing steps and ammonia produced by said upgrading step, the nitrogen fixing cyanobacteria naturally occurring in a type of soil to which the biofertilizer is to be applied; and (e) producing a biofertilizer incorporating the cyanobacteria.

Embodiments provide a highly efficient integrated coal and biomass to liquids (ICBTL) process scheme for producing premium fuels such as gasoline, diesel, jet fuel, and chemical feedstocks, that makes beneficial use of generated C0₂ involving four major process steps:
1 - C0₂ carbon capture and conversion to a biological material such as algae;
2 - direct coal liquefaction (DCL);
3 - indirect conversion of biomass and/or coal to liquid fuels, e.g., by gasification and Fischer Tropsch conversion or by catalytic hydrodeoxygenation and isomerization (CHI); and
4 - hydrogen generation, e.g., by steam hydrogasification (SHG) or POX of the bottoms from the coal conversion, by steam methane reforming (SM R) of a feed such as natural gas, or via the water-gas shift reaction. Alternatively, the hydrogen can be supplied from an external source. Combustible waste streams from the different components of the system may be used to produce electrical power for internal use in the system or for supply to the electrical power grid.

The molybdenum containing catalyst may be produced in situ from a PMA catalyst precursor, and wherein phosphorus obtained from said catalyst precursor is supplied to said photobioreactor as a nutrient.

The hydrogen producing step includes gasifying said carbonaceous feed in a partial oxidation reactor or a gasifier and wherein said carbonaceous feed includes bottoms from said direct liquefaction step or from said upgrading step or from both. It may include reacting natural gas in a steam methane reformer to produce hydrogen and by-product CO₂.

Bottoms produced by the direct liquefaction step and limestone may be fed to a circulating fluid bed boiler for powering an electric power generation system.

Lipids may be extracted from cyanobacteria produced in said photobioreactor and converted to hydrocarbon liquids. Biomass residues remaining after the extraction of said lipids may be supplied as an input to said hydrogen producing step, and hydrogen produced by said hydrogen producing step may be supplied to the lipid conversion step.

The cyanobacteria being reproduced to produce said inoculant are obtained from biological soil crust. The biofertilizer may further include one or more additional microorganisms selected from the groups comprising free-living nitrogen-fixing heterotropic bacteria, actinomycetes, photosynthetic bacteria, mycorrhizal or lichenizing fungi, and combinations thereof, and symbiotic nitrogen-fixing microorganisms including rhizobacteria. It may be transformed into a dormant state by a technique selected from the group consisting of spray drying, refractance-window drying, solar drying, air drying, or freeze drying.

Xxeroprotectant additives including one or more of sorbitol, mannitol, sucrose, sorbitan monostereate, dimethyl sulfoxide, methanol, beta-carotene, and beta-mercaptoethanol may be used to increase post drying viability. The biofertilizer may be combined with an additive selected from the group comprised of fibrous, cellulosic mulch material, polymeric tackifiers, clays, geotextiles, and combinations thereof.

Advantageously, the byproduct CO₂ carbon capture and conversion involves the use of the CO₂ to produce microorganisms including algal biomass such as cyanobacteria and preferably other photosynthetic microorganisms in a closed photobioreactor (PBR). The microorganisms may be used as all or part of the biomass used in the indirect conversion to produce additional liquid fuels. In that case, preferably, the algal biomass is first processed to extract the lipids that can be directly converted into fuels, e.g., by catalytic hydrodeoxygenation and isomerization, and the residual material is used as a feed to the indirect conversion process. More preferably, microorganisms produced by the PBR is used in a biofertilizer or soil amendment, in which case the microorganisms include cyanobacteria, and preferably other photosynthetic microorganisms, that impart beneficial properties to the soil to which the biofertilizer is applied.

The generation of algal biomass and photosynthetic microorganisms to produce fertilizer may be maximized in order to achieve the greatest reduction in lifecycle GHG footprint for associated processes, such as power generation. In this embodiment, the process preferably involves direct coal liquefaction to produce liquid fuels after product separation and upgrading, with the bottoms from the liquefaction and additional coal being used to generate hydrogen and CO₂ in a POX system. The CO₂ is used to produce a biofertilizer. Alternatively, instead of direct coal liquefaction and POX to generate the CO₂ for producing algal biomass and photosynthetic microorganisms for use as biofertilizer, the CO₂ can be generated in part or totally from natural gas in an SMR. The H₂ produced by the SMR is supplied to the coal liquefaction and liquid upgrading steps.

After inoculation of soil with a cyanobacteria-based biofertilizer, the algal microorganisms repopulate the soil through natural reproduction, using sunlight, and nitrogen and CO₂ from the atmosphere, at much higher concentration than originally applied to the soil, thereby substantially reducing, or even eliminating, the CO₂ footprint of the overall ICBTL (integrated coal and biomass to liquids) process on a lifecycle basis and substantially increasing the fertility of the soil for plant growth.

Preferably the biofertilizer includes a soil inoculant cultured from the set of microorganisms including cyanobacteria, also called blue-green algae, and, preferably, other photosynthetic microorganisms, that are already present in the soil or type of soil to which the biofertilizer is to be applied. The biofertilizer soil application rates can range from one gram per square meter to greater than 25 grams per square meter depending on soil type and soil moisture. This provides a highly leveraged effect on soil (terrestrial) carbon sequestration and greatly increases the fertility of the soil. Starting with one ton of DCL process CO₂, the application of the biofertilizer can result, on a lifecycle basis, in several tens of tons of additional CO₂ being removed from the atmosphere and sequestered in the treated soil and in vegetation, crops and/or trees grown in the soil.

In accordance with a still further aspect of the invention, during times such as cloudy days or at night when there is not enough available ambient sunlight to drive the photosynthesis for producing algal biomass and photosynthetic microorganisms, CO₂ produced by the ICBTL process of the invention is stored until sunlight is available, e.g., by liquefying the CO₂ or by storing it under pressure in bladders that can be part of or adjacent to the PBRs being used to produce the algal biomass and photosynthetic microorganisms. Alternatively, it is also possible to illuminate the contents of the PBR during non-sunlit hours in order to maintain the productivity of the algal biomass and photosynthetic microorganisms.

Important advantageous synergies in the ICBTL process and system that contributed substantially to its overall efficiency and economic attractiveness include the facts that the CO₂ stream produced by the gasification and/or SM R is highly concentrated and an ideal feed for producing algal biomass and photosynthetic microorganisms, and that the NH₃ inherently produced in the direct liquefaction and upgrading steps is an important nutrient in the algal biomass and photosynthetic microorganisms production step. Phosphorus, which is also a nutrient in algal biomass production, can be isolated from the PMA catalyst precursor used in the DCL step. Also, oxygen produced in the production of algal biomass can be supplied to the POX system.

Fuels and fuel blends stocks produced by DCL contain high concentrations of cycloparaffins and aromatics. The indirect conversion, on the other hand, produces fuels or fuel blends stocks that are high in isoparaffins that make very high Cetane diesel fuels and can be used as blendstocks for producing jet fuels such as JP8.

In accordance with a second embodiment having extremely high thermal efficiency, low GHG footprint and substantially lower cost than processes involving indirect liquefaction, the process involves direct coal liquefaction to produce, after product separation and upgrading, liquid fuels such as LPG, gasoline, jet fuel and diesel. Additional hydrogen is supplied to the coal liquefaction and product upgrading reactors. Such additional hydrogen can be generated, e.g., by reacting natural gas in a steam methane reformer (SMR). Bottoms from the direct coal liquefaction reactor are preferably fed to a circulating fluid bed (CFB) boiler for use in an electrical power generating system. CO₂ produced by the SMR are preferably supplied to the PBR to produce algal biomass and preferably other photosynthetic microorganisms for use as a biofertilizer, or as a feedstock for other processes. Most preferably, the algal biomass and photosynthetic microorganisms are used as a biofertilizer or soil amendment.

Divisional application EP 18 179 775.4 as filed describes a method for producing a biofertilizer comprising the steps of: (a) inoculating a photobioreactor (PBR) with the naturally occurring complement of microorganisms, including cyanobacteria, occurring in the soil or type of soil to which the biofertilizer is to be applied, or with microorganisms that includes one or more cyanobacteria strains and optionally other photosynthetic microorganisms suitable for use as a fertilizer in the location where the biofertilizer is to be used; (b) amplifying such microorganisms in a photobioreactor (PBR) fed with sunlight, nutrients, and carbon dioxide; (c) harvesting the produced biomass; (d) dewatering and drying the harvested biomass whilst preserving it so that the cells remain viable; and (e) pulverizing, flaking, or powdering the dried biomass to facilitate packaging, storage, shipment, and final dissemination.

The naturally occurring complement of microorganisms is drawn from its normal residence in the top centimeter of healthy undisturbed soil found in un-shaded areas having similar soil and environmental characteristics as the soil to which the biofertilizer is to be applied. The divisional application also claims a method for producing a biofertilizer comprising the steps of: (a) inoculating a photobioreactor (PBR) with inoculant including nitrogen fixing cyanobacteria naturally occurring in a type of soil to which the biofertilizer is to be applied; (b) supplying CO₂ to said PBR for reproducing the inoculated cyanobacteria therein; and (c) incorporating the reproduced cyanobacteria in the biofertilizer.

### BRIEF DESCRIPTION OF THE DRAWINGS

FIG. 1 is a simplified flow chart of one embodiment of an integrated coal and biomass-to- liquids system;
FIG. 2 is a schematic diagram of a direct coal liquefaction system suitable for use in the illustrated embodiments of such a system.
FIG. 3 is a simplified flowchart of another embodiment involving direct coal liquefaction and fertilizer production from algal biomass and photosynthetic microorganisms.
FIG. 4 is a simplified flowchart of another embodiment involving direct coal liquefaction and increased production of fertilizer from algal biomass and photosynthetic microorganisms.

### DETAILED DESCRIPTION OF EMBODIMENTS

In accordance with a first embodiment of the ICBTL process and system, coal is converted by DCL to liquids, and biomass and/or additional coal is converted to liquids by indirect liquefaction by means of biomass hydrodeoxygenation, or by Fischer Tropsch conversion of syngas produced by the gasification of DCL heavy residues, biomass residues and/or coal or coal wastes. The liquids produced by the direct and indirect liquefaction steps are upgraded to produce premium fuels such as gasoline, diesel and jet fuel, and chemical feedstocks. Optionally, as an alternative, natural gas may be reacted by SM R, to produce the syngas for the indirect liquefaction process step. The gasification or SM R also produces additional hydrogen for the DCL, indirect liquefaction and upgrading steps.

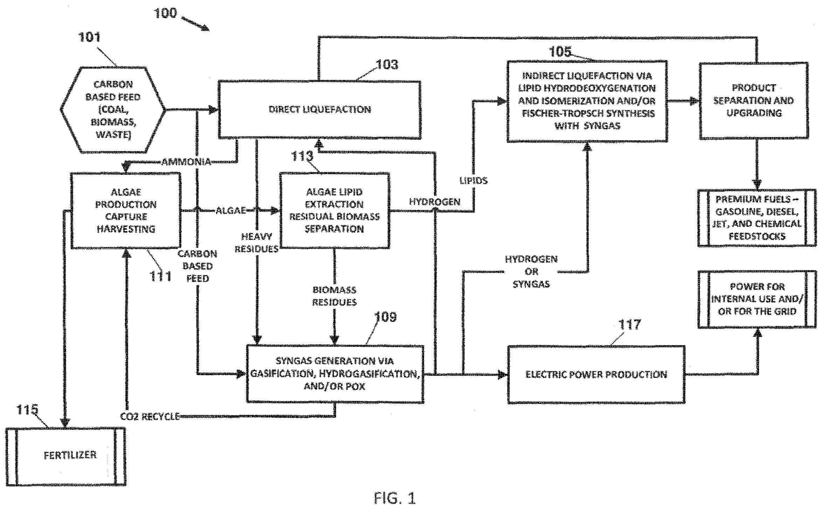

Referring to the embodiment of the ICBTL system 100 illustrated in FIG. 1 of the drawings, carbon-based feed from source 101, which feed includes coal, and may also include biomass and waste coal, is supplied to the DCL reactor system 103, and optionally to the hydrogen and syngas generating system 109. The feed is liquefied in the DCL reactor system 103 by heating in the presence of a solvent and optionally a catalyst. The produced liquids are isolated and upgraded in the product separation and upgrading system 107 to produce premium fuels, such as gasoline, diesel and jet, and chemical feedstocks. The heavy residues from the DCL reactor system 103 are fed to the syngas generating system 109, which may be any one of a variety of conventional systems such as a gasifier, a POX reactor or a hydro-gasification reactor. CO₂ produced by the syngas generating system 109, and optionally, by the DCL reactor system 103, the indirect liquefaction system 105 and/or other components of the ICBTL system, is fed to the algae production system 111, which preferably includes a closed PBR in which the CO₂ is used to produce blue-green algae through photosynthesis. The DCL reactor system 103 and upgrading system 107 also produce N H₃, which is fed to the algae production system 111 as a nutrient. Phosphorus can also be recovered from the PMA catalyst precursor and used as a nutrient for the algae production if the DCL reactor system incorporates the use of a molybdenum catalyst.

Tail gas from the FT synthesis, which includes unreacted hydrogen and CO, can be supplied to the input of the DCL for supplying additional reactants. The algae from the algae production system 111 is preferably used to produce a biofertilizer 115, and optionally, as all or part of any biomass being gasified for indirect liquefaction.

Between 0 and 100% of the algae produced by system 111 is fed to the reactor system 113 in which the lipids are extracted from the algae and fed to the indirect liquefaction system 105, and the residual algae biomass is fed to the syngas generation system 109. Exemplary methods and systems for hydro-processing lipids extracted from or produced by algae are disclosed in the published U.S. patent application US 2009/0077864 A1, the contents of which are hereby incorporated by reference. The portion of the algae not supplied to the reactor 113, preferably all or the major part of the algae, is made into natural bio-fertilizer 115.

Hydrogen produced by the syngas generation system 109 is supplied to the DCL reactor system 103. Hydrogen and/or syngas produced by the generation system 109 is also fed to the indirect liquefaction system 105. Hydrogen and/or combustible residual materials from the syngas generating system 109 is fed to the electric power production system 117 where it is used to produce electric power for components of the ICBTL system 100 and/or for supply to the electrical power grid.

### Direct Coal Liquefaction

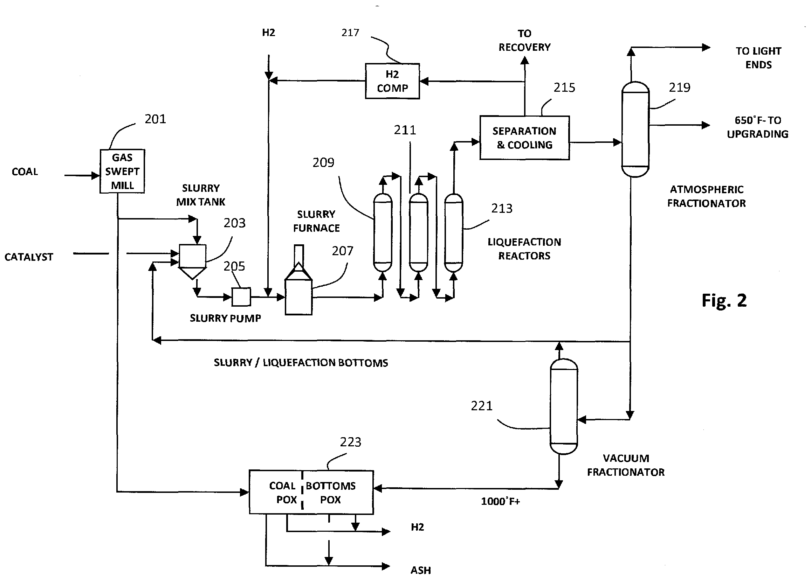

An illustrative embodiment of a reactor system suitable for performing the direct coal liquefaction is shown in Fig. 2 of the drawings. The coal feed is dried and crushed in a conventional gas swept roller mill 201 to a moisture content of 1 to 4 %. The crushed and dried coal is fed into a mixing tank 203 where it is mixed with a solvent containing recycled bottoms and a catalyst precursor to form a slurry stream. The catalyst precursor in the illustrated embodiment preferably is in the form of a 2-10% aqueous water solution of phosphomolybdic acid (PMA) in an amount that is equivalent to adding between 50wppm and 2 % molybdenum relative to the dry coal feed. I n the slurry mix tank 203, the contents are agitated for about 10 to 100 minutes and preferably for 20 to 60 minutes at agitator speed defined a priori as a function of the slurry rheology. Similarly, the operating temperature is set to reflect the same rheological considerations. Typical operating temperature ranges from 250 to 600^ F and more preferably between 300 and 450^ F. From the slurry mix tank the catalyst containing slurry is delivered to the slurry pump 205. The selection of the appropriate mixing conditions is based on experimental work quantifying the rheological properties of the specific slurry blend being processed.

The slurry leaves the mixing tank 203 at about 300 to 500° F (139 to 260° C). Most of the moisture in the coal is driven off in the mixing tank due to the hot recycle solvent (650/1000° F or 353/538° C) and bottom feeding to the mixing tanks. Residual moisture and any entrained volatiles are condensed out as sour water (not shown in Fig. 2). The coal in the slurry leaving the mixing tank 203 has about 0.1 to 1.0% moisture. The slurry formed by the coal and recycled bottoms fraction from the fractionators 219 and 221 is pumped from the mixing tank 203 and the pressure raised to about 2,000 to 3,000 psig (138 to 206 kg/cm² g) by the slurry pumping system 205. The resulting high pressure slurry is preheated in a heat exchanger (not shown), mixed with hydrogen, and then further heated in furnace 207.

The coal slurry and hydrogen mixture is fed to the input of the first stage of the series- connected liquefaction reactors 209, 211 and 213 at about 600 to 700° F (343° C) and 2,000 to 3,000 psig (138 to 206 kg/cm² g). The reactors 209, 211 and 213 are upflow tubular vessels, the total length of the three reactors being 50 to 150 feet. The temperature rises from one reactor stage to the next as a result of the highly exothermic coal liquefaction reactions. In order to maintain the maximum temperature in each stage below about 850 to 900 ° F (454 to 482 ° C), additional hydrogen is preferably injected between reactor stages. The hydrogen partial pressure in each stage is preferably maintained at a minimum of about 1,000 to 2,000 psig (69 to 138 kg/cm² g).

The effluent from the last stage of liquefaction reactor is separated into a gas stream and a liquid/solid stream, and the liquid/solid stream let down in pressure, in the separation and cooling system 215. The gas stream is cooled to condense out the liquid vapors of naphtha, distillate, and solvent. The remaining gas is then processed to remove H₂S and CO₂

Most of the processed gas is then sent to the hydrogen recovery system 17 for further processing by conventional means to recover the hydrogen contained therein, which is then recycled to be mixed with the coal slurry. The remaining portion of the processed gas is purged to prevent buildup of light ends in the recycle loop. Hydrogen recovered therefrom is used in the downstream hydro-processing upgrading system. The depressurized liquid/solid stream and the hydrocarbons condensed during the gas cooling are sent to the atmospheric fractionator 219 where they are separated into light ends, naptha, distillate and bottoms fractions. The light ends are processed to recover hydrogen and C₁-C₄ hydrocarbons that can be used for fuel gas and other purposes. The naphtha is hydrotreated to saturate diolefins and other reactive hydrocarbon compounds. The 160°F + fraction of the naphtha can be hydrotreated and powerformed to produce gasoline. The distillate fraction can be hydrotreated to produce products such as diesel and jet fuel.

The atmospheric fractionator 219 is preferably operated at a high enough pressure so that a portion of the 600 to 700° F+ (315 to 371° C.+) bottoms fraction can be recycled to the slurry mixing tank 203 without pumping for use as the solvent. Pumping of this stream would be difficult because of its high viscosity and high solids content.

The remaining bottoms produced from the atmospheric fractionator 219 are fed to the vacuum fractionator 221 wherein it is separated into of 1000°F- fraction and a 1000°F+ fraction. The 1000°F- fraction is added to the solvent stream being recycled to the slurry mix tank 203. The 1000° F. + fraction is fed to the bottoms partial oxidation gasifier 223 where it is reacted with oxygen to produce hydrogen and CO₂ by means of partial oxidation and water-gas shift reactions. If additional hydrogen is needed for the direct coal liquefaction and upgrading of the products thereof, a portion of the coal from the gas swept roller mill 201 is fed to the coal partial oxidation gasifier 225 for producing the additional required hydrogen. The ash resulting from the partial oxidation of the 1000° F. + fraction and of the coal in the gasifiers 223 and 225 can be can be sent to the landfill or can be used to produce construction materials such as cement bricks, road surface paving material and other construction applications.

If the coal being converted by DCL is lignite, which has a higher H₂0 and 0₂ content than bituminous or sub-bituminous coal, it is preferred to pre-treat the coal in an aqueous carbon monoxide- containing environment, as described in U.S. 5,026,475, the disclosure of which is hereby incorporated by reference in its entirety.

If the DCL process is being operated with relatively low catalyst concentrations of about 50 wppm to 500 wppm, in which about 70 to 80% of the input coal is converted to products, it is economically preferable to recycle only the portions of the catalyst that are entrained in the solvent stream being fed back to the slurry mix tank 203. At higher catalyst concentrations of about 1 to 5 wt%, in which about 80 to 95% of the input coal is converted to products, it is preferred to recover the remaining catalyst from the ash produced by the bottoms partial oxidation 223 by a process such as the one described in U .S. Patent 4,417,972, the disclosure of which is hereby incorporated by reference in its entirety.

Catalysts useful in DCL processes also include those disclosed in U.S. Patents Nos. 4,077,867, 4,196,072 and 4,561,964, the disclosures of which are hereby incorporated by reference in their entirety.

Other DCL processes and reactor systems suitable for use in the ICBTL system 100 are disclosed in U .S. Patents Nos. 4,485,008, 4,637,870, 5,200,063, 5,338,441, and 5,389,230, the disclosures of which are hereby incorporated by reference in their entirety.

Referring again to Fig. 1 of the drawings, an exemplary process for upgrading the liquid product of the DCL 103 is disclosed in U.S. Patent number 5,198,099, the disclosure of which is hereby incorporated by reference in its entirety. Other processes and systems suitable for upgrading the liquid products of the DCL 103 and the indirect liquefaction 105 are commercially available from vendors such as UOP, Axems, Criterion and others. If the syngas generation system 109 (Fig. 1) includes POX reactor, one of a variety of commercially available POX systems may be used. During partial oxidation, in processes provided commercially by Shell, G.E., Siemens and others, nitrogen compounds in the coal are converted principally to N₂. Oxygen in the coal is converted to CO, CO₂, and a small amount of COS. Sulfur is converted to H₂S and HCN. The product gas is cooled and cleaned to remove particulates and other gases, leaving only CO, CO₂, and H₂. If this stream is to be used in DCL or upgrading, it is then reheated and sent to a water- gas shift section where CO and H₂0 are converted to H₂ and CO₂ in the presence of a catalyst. The gas from the water gas shift reactor, which contains H₂S, CO₂, and H₂ for use in DCL or a mixture of H₂S, CO₂, CO, and H₂ for F-T, is then sent to a separation system such as Rectisol or Selexol. These processes are offered commercially by UOP, and others. During this step, separate H₂ or H₂/CO, H₂S, and CO₂ streams are produced. One key advantage of Selexol is that it produces the CO₂ at higher pressure than scrubbing processes such as MEA. This reduces the quantity of compression required to store the CO₂ or to transport the CO₂ to the algae production system 111. The H₂S and COS, once hydrolyzed, are removed by dissolution in, or reaction with, an organic solvent and converted to valuable by-products such as elemental sulfur or sulfuric acid.

The raw synthesis gas must be reheated before entering a conventional water gas shift reactor system that produces additional hydrogen through the catalytically assisted equilibrium reaction of CO and H₂0 to form CO₂ and H₂. Hydrogen is then separated from the CO₂, CO, and other contaminants and undergoes a final polishing step prior to being sent to liquefaction or upgrading. Minerals in the coal (ash) separate and leave the bottom of the gasifier as an inert slag. The fraction of the ash entrained with the syngas is removed downstream in filters or water scrubbers. This material is typically recycled to the gasifier.

### FT Synthesis

The indirect liquefaction system 105 can be implemented using Fischer Tropsch reactor system. Reactors, catalysts and conditions for performing FT synthesis are well known to those of skill in the art and are described in numerous patents and other publications, for example, in U.S. Patents Nos.7,198,845, 6,942,839, 6,315,891, 5,981608 and RE39,073, the contents of which are hereby incorporated by reference in their entirety. FT synthesis can be performed in fixed bed, moving bed, fluid bed, ebulating bed or slurry reactors using various catalysts and under various operating conditions that are selected based on the desired product suite and other factors. Typical FT synthesis products include paraffins and olefins, generally represented by the formula nCH₂. The productivity and selectivity for a given product stream is determined by reaction conditions including, but not limited to, reactor type, temperature, pressure, space rate, catalyst type and syngas composition.

The stoichiometric syngas H₂/CO ratio for FT synthesis is about 2.0. The ratio of H₂/CO in syngas produced from coal is less than 2, and typically about 0.5. This ratio can be increased by mixing the coal produced syngas with syngas produced from biomass or natural gas, or by producing the syngas from a mixed coal and biomass feed. If such mixing step does not increase the H₂/CO ratio adequately, and additional hydrogen is not conveniently available from other sources, such ratio may be further increased by the water-gas shift reaction. In the case of FT synthesis conversion performed using a cobalt-based catalyst, which does not a promote water-gas shift reaction, the H₂/CO ratio of coal produced a syngas is preferably increased to about 2.0 before being introduced in the FT synthesis reactor, e.g., by hydrogen produced by the syngas generating system 109. If the FT synthesis conversion is being performed using an iron- based catalyst, which does provoke the water-gas shift reaction, it is not necessary to use a separate shift converter. In any case, however, the water-gas shift reaction generates additional CO₂.

### Hvdrodeoxygenation

If the feed to the indirect liquefaction system 105 consists entirely of biomass, such as lipids extracted from algae and/or other biomass sources, it can alternatively be catalytic hydrodeoxygenation and isomerization implemented using a (CHI) system, or similar systems, such as disclosed in published international applications WO 2009/025663, WO2009/025635, WO2008/8124607 or US Patent No.4,992,605, the contents of which are hereby incorporated by reference in their entireties.

### CO₂ Capture and Re-use

As described above, CO₂ produced by the above process is preferably captured and used to produce microorganisms including algal biomass and
photosynthetic microorganisms in a PBR. The PBR system can involve closed or open reactor systems; with closed systems being preferred to enable maximum production of specifically selected strain(s) of algal biomass and photosynthetic microorganisms and to minimize water loss and the contamination of the algal biomass and photosynthetic microorganisms from external sources, and to allow the capture of oxygen produced in the algal biomass generation step for use in other combustion or POX related steps in the overall ICBTL process. There are a number of commercially available algal biomass and photosynthetic microorganism production systems. One preferred system is the closed PBR system described in published US patent application numbers 2007/0048848 and 2007/0048859, which are incorporated herein by reference in their entirety.

The algal biomass and photosynthetic microorganisms produced in the PBR can be isolated in aqueous streams for use as a soil treatment material in order to increase the carbon content of the soil and for inducing photosynthesis to self-replicate in the soil. The resulting microorganisms can also be dried and combined with other additives such as organic binders, alkali containing residues from the gasification and/or DCL facility and the final mixture used as a natural bio-fertilizer. I n this capacity, the material not only results in further growth of such microorganisms in the soil via photosynthesis, thereby increasing its natural carbon content, but also causes various components of the algal biomass (e.g. cyanobacteria) and other microorganisms to fix nitrogen, all of which promotes the growth of plant life in the treated soil and greatly reduces the GHG, and particularly the CO₂, footprint of the ICBTL process. PBR systems suitable include those described in provisional US patent application, Serial No. 61/422,613, subsequently published as Statutory Invention Registration H2,271, and those developed by BioProcess Algae, LLC, Phyco Biosciences, or Solix BioSystems.

In accordance with a preferred embodiment, the naturally occurring complement of microorganisms, including cyanobacteria, occurring in the soil or type of soil to which the biofertilizer is to be applied is optimized and amplified in a closed PBR and the resulting material is dewatered and dried and treated with desirable additives; after which it is granulated, optionally coated with materials to optimize its spreading characteristics and distributed on the soil that is to be fertilized or restored. Alternatively, microorganisms that include one or more strains of cyanobacteria and other components compatible with the type of soil and environmental conditions where the biofertilizer is to be applied, are amplified in a closed PBR to generate the material for the biofertilizer.

In addition to the beneficial reduction of the GHG footprint of the ICBTL by terrestrially sequestering the CO₂ consumed by cyanobacteria in the produced fertilizer, the integrated system has the additional extremely important advantageous characteristic that the set of cyanobactraia and other microorganisms applied to the soil especially because it can be specifically selected to be compatible with the makeup of the soil to which it is applied, multiplies, e.g., through photosynthesis, thereby extracting more CO₂ from the atmosphere and fixing atmospheric nitrogen. This characteristic results in an increase in the net CO₂ sequestered by a factor of 30 and potentially as much 150-fold over the CO₂ consumed during the production of the microorganisms in the ICBTL process, and greatly enriches the fertility of soil. The biofertilizer can also be mixed with the soil as a soil amendment.

The quality of the natural bio-fertilizer, as affected by the quality of the water and the purity of the CO₂ and other nutrient streams provided to the PBR from other steps in the ICBTL process, can be controlled to generate food grade/FDA certified material for use in enhancing growth of various food crops; to an intermediate grade to serve as a soil amendment material for reclamation of arid soils to prevent or inhibit wind erosion via formation of a bio-active crust; or to lower purity material for use in reclamation of spent mine soils where the addition of a bio-reactive material inhibits leaching and erosion of contaminated soils to improve the quality of water drain off.

The natural bio-fertilizer can also be used as a direct replacement for conventional ammonia-based fertilizer, where it offsets large amounts of CO₂ that would otherwise be generated in production of NH₃ and the full range of ammonia based fertilizers. This also leads to other downstream benefits, such as a reduction in run off of NH₃ based components that contaminate downstream waterways and cause unwanted blooms of algae and other aquatic plants.

In order to minimize the CO₂ footprint in the system and convert substantially all of the CO₂ to algae, the CO₂ can be stored during periods of low light or darkness when there is not enough light for photosynthesis to produce algal biomass and photosynthetic microorganisms from the CO₂. Alternatively, microorganism production can be continued using artificial light sources. To further minimize the CO₂ footprint on a lifecycle basis, the algae is then used to produce a bio-fertilizer. Coupling these steps together allows for recovery and reuse of the equivalent of as much as 270 times the CO₂ conversion to algae alone using an open pond or PBR without the use of artificial light. Without storage, the quantity of CO₂ reused is reduced by a factor of three or four. Techniques for storage of CO₂ include liquefaction of the CO₂, conversion of the CO₂ to ammonium bisulfide or urea by well-known conventional chemical processes, physical storage and others.

Thus, a preferred embodiment of the ICBTL process and system involves an integrated process sequence that comprises several different processing steps:
1. Gasification of carbon containing feeds to produce hydrogen or synthesis gas.
2. Direct liquefaction of coal to produce a series of distillate range hydrocarbons.
3. Capture of process CO₂ to produce algae in a PBR.
4. Isolation of the algae in aqueous solution or in a dried state.
5. Use of algae in one or more of three separate steps including: (a) a feedstock to step 1 above; (b) as a feed to a lipid recovery step to generate triglyceride fatty acids (TGFA) and/or free fatty acids (FA); (c) as a natural fertilizer or soil treatment material to enhance or facilitate terrestrial sequestration of CO₂.
6. Conversion of some or all of the produced TGFA or FA to generate isoparaffinic distillates via Catalytic Hydrodeoxygenation and Isomerization (CHI).
7. Optional or alternative use of synthesis gas to produce normal paraffins via Fischer Tropsch Synthesis (FT).
8. Optional or alternative use of hydroisomerization to convert normal paraffins to iso-paraffins.
9. Upgrading of coal derived liquids form step 2 to generate heteroatom free aromatics and/or cycloparaffinic hydrocarbons in the distillate fuel range.

There are several commercial systems available for separating hydrogen from carbon monoxide. Pressure swing adsorption (PSA) processes rely on the fact that under pressure, gases tend to be attracted to solid surfaces, or "adsorbed". The higher the pressure, the more gas is adsorbed; when the pressure is reduced, the gas is released, or desorbed. PSA processes can be used to separate gases in a mixture because different gases tend to be attracted to different solid surfaces more or less strongly. Syngas mixtures of H₂, CO and CO₂ can be separated by PSA to produce streams rich in hydrogen. Alternatively, syngas ca n be first subjected to water gas shift to produce a binary mixture of H₂ and CO₂ which can be separated by PSA or by other means known in the art such as membrane separation (where H₂ permeates much more effectively than CO₂ to generate substantially pure hydrogen streams). Finally, active metal membranes of palladium and other related metal alloys may be used to separate hydrogen from other gases and commercially available options have been produced. U.S. Patents Nos. 5,792,239, 6,332,913 and 6,379,645, and published applications Nos. US2003/3190486 and US2009/0000408 describe various ones of such separation techniques and are hereby incorporated by reference in their entireties.

The CO₂ recovery can be conducted using various conventional recovery processes including, but not limited to, adsorption, absorption (e.g. pressure swing adsorption (PSA) and displacement purge cycles (DPC)), cryogenic separation, membrane separation, combinations thereof and the like. While one or more recovery processes may be needed to recover CO₂ from syngas or tail gas, by-product gas from a reformer or C3+ product upgrader will not contain appreciable amounts of H₂ or H₂0 and thus may not need any recovery process except for condensation of heavy hydrocarbons (C6+). Additionally, while it is desirable to use recovered CO₂, it is also possible to supplement or replace recovered CO₂ with CO₂ obtained from alternative sources within an integrated complex.

Product streams from the process can include, for example, a synthetic crude and other individual product streams such as liquefied petroleum gas (C3- C4), condensate (C5-C6), high-octane blend components (C6-C10 aromatic-containing streams), jet fuel, diesel fuel, other distillate fuels, lube blend stocks or lube blend feedstocks that can be produced and sold as separate products.

The fully integrated process flow scheme illustrated in Fig.1 provides a combination of features and advantages that cannot be achieved with known alternatives. The process combines direct and indirect conversion together with on-site CO₂ capture and conversion to liquid fuel components and generates over 4 barrels of clean burning liquid fuel per ton of coal. This is approximately twice the liquid yield that is possible when compared to other technologies now being offered.

Novel process integration also enables the more effective utilization of by-product streams from one section of the ICBTL facility as feedstocks for another. This superior design improves overall efficiency and eliminates a critical barrier to entry by reducing overall investment by 15-20%, thereby allowing the generation of nearly twice the value per ton of coal versus alternative coal to liquids routes.

### Upgrading and Products

By blending DCL liquids with algae-derived liquids, the resulting fuel will be significantly below petroleum fuel in carbon footprint and due to its unique composition, it will have better performance properties. Synthetic JP8, JP7, JP5, JP9 and rocket fuel production with the ICBTL process is able to produce fuel mixtures comprising selected aromatics, cycloparaffins and isoparaffins - together with specific amounts of various linear paraffin molecules. Jet fuel for military use falls into several different categories where the physical and chemical properties of the fuel are varied to control flash point, freeze point, materials compatibility properties such as corrosion in copper clad distribution equipment, and other properties related to thermal management and heat transfer or to total energy content as will ultimately determine hover time or range on fuel load, e.g., for unmanned combat aerial vehicles. The ICBTL system of the illustrated embodiment produces individual streams of those classes of molecules - and blending can be done in ways to produce individual fuels as well as more conventional blends, including those to meet Jet Al and synthetic ASTM D7566 standards.

The isoparaffins produced in ICBTL can be tailored to have controlled branching density and branching index - to ultimately control the overall thermal and oxidative stability of the products.

The degree of hydroprocessing coupled with continuous monitoring of product structure by on-line GC-MS or 13C NMR allows process conditions to be tailored to the desired average structural composition. The extent of branching and branching position can be determined by NMR Analysis.

### NMR Branching Analysis

The branching properties of the hydrocarbon distillates and intermediate isomerates may be determined by analyzing a sample of distillate using carbon-13 NMR according to the following eight-step process. References cited in the description of the process provide details of the process steps. Steps 1 and 2 are performed only on the initial materials from a new process.
1. Identify the CH branch centers and the CH₃ branch termination points using the DEPT Pulse sequence (Doddrell, D. T.; D. T. Pegg; M. R. Bendall, Journal of Magnetic Resonance 1982, 48, 323ff.).
2. Verify the absence of carbons initiating multiple branches (quaternary carbons) using the APT pulse sequence (Patt, S. L; J. N. Shoolery, Journal of Magnetic Resonance 1982, 46, 535ff.).
3. Assign the various branch carbon resonances to specific branch positions and lengths using tabulated and calculated values (Lindeman, L. P., Journal of Qualitative Analytical Chemistry 43, 19711245ff; Netzel, D. A., et. al., Fuel, 60, 1981, 307ff.).
4. Quantify the relative frequency of branch occurrence at different carbon positions by comparing the integrated intensity of its terminal methyl carbon to the intensity of a single carbon (=total integral/number of carbons per molecule in the mixture).
   For the unique case of the 2-methyl branch, where both the terminal and the branch methyl occur at the same resonance position, the intensity was divided by two before doing the frequency of branch occurrence calculation. If the 4-methyl branch fraction is calculated and tabulated, its contribution to the 4+ methyls must be subtracted to avoid double counting.
5. Calculate the Free Carbon Index using the calculated average carbon number of the sample and the results from the C-13 NM R analysis, as described in EP 1062305. The Free Carbon Index (FCI) is a measure of the number of carbon atoms in an isoparaffin that are located at least 5 carbons from a terminal carbon and 4 carbons away from a side chain. The average carbon number may be determined with sufficient accuracy for lubricant materials by dividing the molecular weight of the sample by 14 (the formula weight of CH2). Molecular weight may be determined by ASTM D2502, ASTM D2503, or other suitable method. Molecular weight is preferably determined by ASTM D2503-02.
6. Calculate the Branching Index (Bl) and Branching Proximity (BP) using the calculations described in U.S. Pat. No. 6,090,989. Branching Index is the ratio in percent of non-benzylic methyl hydrogens in the range of 0.5 to 1.05 ppm, to the total non-benzylic aliphatic hydrogens in the range of 0.5 to 2.1 ppm. The Branching Proximity is the % equivalent recurring methylene carbons, which are five or more removed from an end group or branch (epsilon carbons).
7. The number of branches per molecule is the sum of the branches found in step 4.
8. The number of alkyl branches per 100 carbon atoms is calculated from the number of branches per molecule (step 7) times 100/number of carbons per molecule. Measurements can be performed using any Fourier Transform N M R spectrometer. Preferably, the measurements are performed using a spectrometer having a magnet of 7.0 T or greater. In all cases, after verification by Mass Spectrometry, UV or an N M R survey that aromatic carbons were absent, the spectral width was limited to the saturated carbon region, about 0 80 ppm vs. TMS (tetramethylsilane). Solutions of 15 25% by weight in chloroform-dl were excited by 45 degree pulses followed by an 0.8 sec acquisition time. In order to minimize non-uniform intensity data, the proton decoupler was gated off during a 10 sec delay prior to the excitation pulse and on during acquisition. Total experiment times ranged from 11 80 minutes. The DEPT and APT sequences were carried out according to literature descriptions with minor deviations described in the Varian or Bruker operating manuals.

### Hydrocarbon Upgrading to Control Average Molecular Composition

Hydroisomerization can be conducted using a shape selective intermediate pore size molecular sieve. Hydroisomerization catalysts useful for this purpose comprise a shape selective intermediate pore size molecular sieve and optionally a catalytically active metal hydrogenation component on a refractory oxide support. The phrase "intermediate pore size," as used herein means an effective pore aperture in the range of from about 4.0 to about 7.1 ANG. when the porous inorganic oxide is in the calcined form. The shape selective intermediate pore size molecular sieves used are generally 1-D 10-, 11- or 12-ring molecular sieves.

Preferred molecular sieves are of the 1-D 10-ring variety, where 10-(or 11-or 12-) ring molecular sieves have 10 (or 11 or 12) tetrahedrally-coordinated atoms (T-atoms) joined by oxygens. I n the 1-D molecular sieve, the 10-ring (or larger) pores are parallel with each other, and do not interconnect. The classification of intrazeolite channels as 1-D, 2-D and 3-D is set forth by R. M . Barrer in Zeolites, Science and Technology, edited by F. R. Rodrigues, L. D. Rollman and C. Naccache, NATO ASI Series, 1984.

Preferred shape selective intermediate pore size molecular sieves used for hydroisomerization are based upon aluminum phosphates, such as SAPO-11, SAPO-31, and SAPO-41. SAPO-11 and SAPO-31 are more preferred, with SAPO-11 being most preferred. SM-3 is a particularly preferred shape selective intermediate pore size SAPO, which has a crystalline structure falling within that of the SAPO-11 molecular sieves. The preparation of SM-3 and its unique characteristics are described in U.S. Pat. Nos. 4,943,424 and 5,158,665. Also preferred shape selective intermediate pore size molecular sieves used for hydroisomerization are zeolites, such as ZSM-22, ZSM-23, ZSM-35, ZSM-48, ZSM-57, SSZ-32, offretite, and ferrierite. SSZ-32 and ZSM-23 are more preferred.

A preferred intermediate pore size molecular sieve is characterized by selected crystallographic free diameters of the channels, selected crystallite size (corresponding to selected channel length), and selected acidity. Desirable crystallographic free diameters of the channels of the molecular sieves are in the range of from about 4.0 to about 7.1 Angstrom, having a maximum crystallographic free diameter of not more than 7.1 and a minimum crystallographic free diameter of not less than 3.9 Angstrom. Preferably the maximum crystallographic free diameter is not more than 7.1 and the minimum crystallographic free diameter is not less than 4.0 Angstrom. Most preferably the maximum crystallographic free diameter is not more than 6.5 and the minimum crystallographic free diameter is not less than 4.0 Angstrom.

A particularly preferred intermediate pore size molecular sieve, which is useful in the present process is described, for example, in U.S. Pat. No.5,135,638 and 5,282,958, the contents of which are hereby incorporated by reference in their entirety. In U.S. Pat. No.5,282,958, such an intermediate pore size molecular sieve has a crystallite size of no more than about 0.5 microns and pores with a minimum diameter of at least about 4.8.ANG. and with a maximum diameter of about 7.1.ANG. The catalyst has sufficient acidity so that 0.5 grams thereof when positioned in a tube reactor converts at least 50% of hexadecane at 370. degree. C, a pressure of 1200 psig, a hydrogen flow of 160 ml/min, and a feed rate of 1 ml/hr. The catalyst also exhibits isomerization selectivity of 40 percent or greater (isomerization selectivity is determined as follows:
100. times. (weight % branched C₁₆ in product)/(weight % branched C₁₆ in product + weight % C₁₃- in product) when used under conditions leading to 96% conversion of normal hexadecane (n-C₁₆) to other species.

Such a particularly preferred molecular sieve may further be characterized by pores or channels having a crystallographic free diameter in the range of from about 4.0 to about 7.1.ANG., and preferably in the range of 4.0 to 6.5.ANG. The crystallographic free diameters of the channels of molecular sieves are published in the "Atlas of Zeolite Framework Types", Fifth Revised Edition, 2001, by Ch. Baerlocher, W. M. Meier, and D. H. Olson, Elsevier, pp 10 15.

If the crystallographic free diameters of the channels of a molecular sieve are unknown, the effective pore size of the molecular sieve can be measured using standard adsorption techniques and hydrocarbonaceous compounds of known minimum kinetic diameters. See Breck, Zeolite Molecular Sieves, 1974 (especially Chapter 8); Anderson et al. J. Catalysis 58, 114 (1979); and U.S. Pat. No. 4,440,871, the pertinent portions of which are incorporated herein by reference. In performing adsorption measurements to determine pore size, standard techniques are used. It is convenient to consider a particular molecule as excluded if does not reach at least 95% of its equilibrium adsorption value on the molecular sieve in less than about 10 minutes (p/po=0.5;25° C). Intermediate pore size molecular sieves will typically admit molecules having kinetic diameters of 5.3 to 6.5 Angstrom with little hindrance.

Hydroisomerization catalysts optionally comprise a catalytically active hydrogenation metal. The presence of a catalytically active
hydrogenation metal leads to product improvement, especially VI and stability. Typical catalytically active hydrogenation metals include chromium, molybdenum, nickel, vanadium, cobalt, tungsten, zinc, platinum, and palladium. The metals platinum and palladium are especially preferred, with platinum most especially preferred. If platinum and/or palladium is used, the total amount of active hydrogenation metal is typically in the range of 0.1 to 5 weight percent of the total catalyst, usually from 0.1 to 2 weight percent, and not to exceed 10 weight percent.

The refractory oxide support may be selected from those oxide supports, which are conventionally used for catalysts, including silica, alumina, silica-alumina, magnesia, titania and combinations thereof.

The conditions for hydroisomerization will be tailored to achieve an isomerized liquid intermediate with specific branching properties, as described above, and thus will depend on the characteristics of feed used. I n general, conditions for hydroisomerization are mild such that the conversion of hydrocarbon materials boiling below about 700° F. is maintained above about 50 to about 80 wt % in producing the intermediate isomerates.

Mild hydroisomerization conditions are achieved through operating at a lower temperature, generally between about 390. degree. F. and 650. degree. F. at a LHSV generally between about 0.5 hr.sup.-l and about 20 hr.sup.-l. The pressure is typically from about 15 psig to about 2500 psig, preferably from about 50 psig to about 2000 psig, more preferably from about 100 psig to about 1500 psig. Low pressure provides enhanced isomerization selectivity, which results in more isomerization and less cracking of the feed, thus producing an increased yield.

Hydrogen is present in the reaction zone during the hydroisomerization process, typically in a hydrogen to feed ratio from about 0.5 to 30 MSCF/bbl (thousand standard cubic feet per barrel), preferably from about 1 to about 10 MSCF/bbl. Hydrogen may be separated from the product and recycled to the reaction zone.

These mild hydroisomerization conditions using the shape selective intermediate pore size molecular sieves produce intermediate isomerates comprising paraffinic hydrocarbon components having specific branching properties, i.e., having controlled amounts of branching overall.

### Analytical Measurement Techniques

### Specific Analytical Test Methods

Brookfield viscosities were measured by ASTM D 2983-04. Pour points were measured by ASTM D 5950-02.

### Wt % Olefins

The Wt % Olefins in the fuels can be determined by proton-NMR by the following steps, A-D:
A. Prepare a solution of 5-10% of the test hydrocarbon in deuterochloroform.
B. Acquire a normal proton spectrum of at least 12 ppm spectral width and accurately reference the chemical shift (ppm) axis. The instrument must have sufficient gain range to acquire a signal without overloading the receiver/ADC. When a 30. degree pulse is applied, the instrument must have a minimum signal digitization dynamic range of 65,000. Preferably the dynamic range will be 260,000 or more.
C. Measure the integral intensities between: 6.0-4.5 ppm (olefin) 2.2-1.9 ppm (allylic) 1.9- 0.5 ppm (saturate)
D. Using the molecular weight of the test substance determined by ASTM D 2503, calculate:
   1. The average molecular formula of the saturated hydrocarbons.
   2. The average molecular formula of the olefins.
   3. The total integral intensity (=sum of all integral intensities).
   4. The integral intensity per sample hydrogen (=total integral/number of hydrogens in formula).
   5. The number of olefin hydrogens (.dbd. Olefin integral/integral per hydrogen).
   6. The number of double bonds (.dbd. Olefin hydrogen times hydrogens in olefin formula/2).
   7. The wt % olefins by proton NMR=100 times the number of double bonds times the number of hydrogens in a typical olefin molecule divided by the number of hydrogens in a typical test substance molecule.

The wt % olefins by proton NMR calculation procedure, D, works best when the percent olefins result is low, less than about 15 wt %. The olefins must be "conventional" olefins; i.e. a distributed mixture of those olefin types having hydrogens attached to the double bond carbons such as: alpha, vinylidene, cis, trans, and trisubstituted. These olefin types will have a detectable allylic to olefin integral ratio between 1 and about 2.5. When this ratio exceeds about 3, it indicates a higher percentage of tri or tetra substituted olefins are present and that different assumptions must be made to calculate the number of double bonds in the sample.

### Aromatics Measurement by HPLC-UV

A method that can be used to measure low levels of molecules with at least one aromatic function in the fuels uses a Hewlett Packard 1050 Series Quaternary Gradient High Performance Liquid Chromatography (HPLC) system coupled with a HP 1050 Diode-Array UV-V is detector interfaced to an HP Chem-station.

Identification of the individual aromatic classes in the fuels can be made on the basis of their UV spectral pattern and their elution time. The amino column used for this analysis differentiates aromatic molecules largely on the basis of their ring-number (or more correctly, double-bond number). Thus, the single ring aromatic containing molecules elute first, followed by the polycyclic aromatics in order of increasing double bond number per molecule. For aromatics with similar double bond character, those with only alkyl substitution on the ring elute sooner than those with naphthenic substitution. Unequivocal identification of the various fuel aromatic hydrocarbons from their UV absorbance spectra can be accomplished recognizing that their peak electronic transitions were all red-shifted relative to the pure model compound analogs to a degree dependent on the amount of alkyl and naphthenic substitution on the ring system. These bathochromic shifts are well known to be caused by alkyl-group derealization of the .pi. -electrons in the aromatic ring. Since few unsubstituted aromatic compounds boil in the fuel range, some degree of red-shift was expected and observed for all of the principle aromatic groups identified. Quantitation of the eluting aromatic compounds was made by integrating chromatograms made from wavelengths optimized for each general class of compounds over the appropriate retention time window for that aromatic. Retention time window limits for each aromatic class were determined by manually evaluating the individual absorbance spectra of eluting compounds at different times and assigning them to the appropriate aromatic class based on their qualitative similarity to model compound absorption spectra. With few exceptions, only five classes of aromatic compounds were observed in highly saturated API Group II and III lubricant base oils.

### HPLC-UV Calibration

HPLC-UV is used for identifying these classes of aromatic compounds even at very low levels. Multi-ring aromatics typically absorb 10 to 200 times more strongly than single- ring aromatics. Alkyl-substitution also affected absorption by about 20%. Therefore, it is important to use HPLC to separate and identify the various species of aromatics and know how efficiently they absorb.

For example, alkyl-cyclohexylbenzene molecules in fuels exhibit a distinct peak absorbance at 272 nm that corresponds to the same (forbidden) transition that unsubstituted tetralin model compounds do at 268 nm. The concentration of alkyl-1- ring aromatic naphthenes in fuels samples can be calculated by assuming that its molar absorptivity response factor at 272 nm is approximately equal to tetralin's molar absorptivity at 268 nm, calculated from Beer's law plots. Weight percent concentrations of aromatics can be calculated by assuming that the average molecular weight for each aromatic class is approximately equal to the average molecular weight for the whole sample.

This calibration method can be further improved by isolating the 1-ring aromatics directly from the fuels via exhaustive HPLC chromatography. Calibrating directly with these aromatics can eliminate the assumptions and uncertainties associated with model compounds.

More specifically, to accurately calibrate the HPLC-UV method, the substituted benzene aromatics can be separated from the bulk of the lubricant base oil using a Waters semi- preparative HPLC unit.10 grams of sample can be diluted 1:1 in n-hexane and injected onto an amino-bonded silica column, a 5 cm. times.22.4 mm ID guard, followed by two 25 cm.times.22.4 mm ID columns of 8-12 micron amino-bonded silica particles, manufactured by Rainin Instruments, Emeryville, Calif., with n-hexane as the mobile phase at a flow rate of 18 mls/min. Column eluent can be fractionated based on the detector response from a dual wavelength UV detector set at 265 nm and 295 nm. Saturate fractions can be collected until the 265 nm absorbance showed a change of 0.01 absorbance units, which signaled the onset of single ring aromatic elution. A single ring aromatic fraction is collected until the absorbance ratio between 265 nm and 295 nm decreased to 2.0, indicating the onset of two ring aromatic elution. Purification and separation of the single ring aromatic fraction is made by re-chromatographing the monoaromatic fraction away from the "tailing" saturates fraction which results from overloading the HPLC column.

### Confirmation of Aromatics by NMR

The weight percent of all molecules with at least one aromatic function in the purified mono-aromatic standard can be confirmed via long-duration carbon 13 NMR analysis. NMR is easier to calibrate than HPLC UV because it simply measured aromatic carbon so the response did not depend on the class of aromatics being analyzed. The NMR results can be translated from % aromatic carbon to % aromatic molecules (to be consistent with HPLC-UV and D 2007) by knowing that 95-99% of the aromatics in highly saturated fuels are single-ring aromatics.

More specifically, to accurately measure low levels of all molecules with at least one aromatic function by N M R, the standard D 5292-99 method can be modified to give a minimum carbon sensitivity of 500: 1 (by ASTM standard practice E 386). A 15-hour duration run on a 400-500 M Hz N M R with a 10-12 mm Nalorac probe can be used. Acorn PC integration software can be used to define the shape of the baseline and consistently integrate. The carrier frequency can be changed once during the run to avoid artifacts from imaging the aliphatic peak into the aromatic region. By taking spectra on either side of the carrier spectra, the resolution can be improved significantly.

### Molecular Composition by FIMS

The fuels produced by this process can be characterized by Field Ionization Mass Spectroscopy (FI MS) into alkanes and molecules with different numbers of unsaturations. The distribution of the molecules in the fuel fractions can be determined by FIMS. The samples can be introduced via solid probe, preferably by placing a small amount (about 0.1 mg.) of the fuel to be tested in a glass capillary tube. The capillary tube can be placed at the tip of a solids probe for a mass spectrometer, and the probe heated from about 40 to 50. degree C up to 500 or 600 degree C at a rate between 50degree C and 1OOdegree C per minute in a mass spectrometer operating at about 10sup-6 torr. The mass spectrometer can be scanned from m/z 40 to m/z 1000 at a rate of 5 seconds per decade.

The mass spectrometer to be used can be a Micromass Time-of-Flight. Response factors for all compound types are assumed to be 1.0, such that weight percent was determined from area percent. The acquired mass spectra can be summed to generate one "averaged" spectrum.

The fuels produced by this process can characterized by FIMS into alkanes and molecules with different numbers of unsaturations. The molecules with different numbers of unsaturations may be comprised of cycloparaffins, olefins, and aromatics. If aromatics were present in significant amounts in the fuels, they would be identified in the FIMS analysis as 4-unsaturations. When olefins are present in significant amounts in the fuel, they would be identified in the FIMS analysis as 1- unsaturations. The total of the 1-unsaturations, 2-unsaturations, 3-unsaturations, 4- unsaturations, 5-unsaturations, and 6-unsaturations from the FIMS analysis, minus the wt % olefins by proton NMR, and minus the wt % aromatics by HPLC-UV is the total weight percent of molecules with cycloparaffinic functionality in the fuels.

Molecules with cycloparaffinic functionality mean any molecule that is, or contains as one or more substituents, a monocyclic or a fused multicyclic saturated hydrocarbon group. The cycloparaffinic group may be optionally substituted with one or more substituents. Representative examples include, but are not limited to, cyclopropyl, cyclobutyl, cyclopentyl, cyclohexyl, cycloheptyl, decahydronaphthalene, octahydropentalene, (pentadecan-6-yl)cyclohexane, 3,7,10-tricyclohexylpentadecane, decahydro-1-(pentadecan-6-yl)naphthalene, and the like.

Molecules with monocycloparaffinic functionality mean any molecule that is a monocyclic saturated hydrocarbon group of 3 to 7 ring carbons or any molecule that is substituted with a single monocyclic saturated hydrocarbon group of 3 to 7 ring carbons. The cycloparaffinic group may be optionally substituted with one or more substituents. Representative examples include, but are not limited to, cyclopropyl, cyclobutyl, cyclopentyl, cyclohexyl, cycloheptyl, (pentadecan-6-yl)cyclohexane, and the like.

Molecules with multicycloparaffinic functionality mean any molecule that is a fused multicyclic saturated hydrocarbon ring group of two or more fused rings, any molecule that is substituted with one or more fused multicyclic saturated hydrocarbon ring groups of two or more fused rings, or any molecule that is substituted with more than one monocyclic saturated hydrocarbon group of 3 to 7 ring carbons. The fused multicyclic saturated hydrocarbon ring group preferably is of two fused rings. The cycloparaffinic group may be optionally substituted with one or more substituents. Representative examples include, but are not limited to, decahydronaphthalene, octahydropentalene, 3,7,10-tricyclohexylpentadecane, decahydro-1-(pentadecan-6-yl) naphthalene, and the like.

### NMR Branching Properties

The branching properties of the fuels can be determined by analyzing a sample using carbon-13 (¹³C) NMR according to the following ten-step process. References cited in the description of the process provide details of the process steps. Steps 1 and 2 are performed only on the initial materials from a new process.
1) Identify the CH branch centers and the CH .sub.3 branch termination points using the DEPT Pulse sequence (Doddrell, D. T.; Pegg, D. T.; Bendall, M . R., Journal of Magnetic Resonance 1982, 48, 323ff .).
2) Verify the absence of carbons initiating multiple branches (quaternary carbons) using the APT pulse sequence (Patt, S. L; Shoolery, J. N ., Journal of Magnetic Resonance 1982, 46, 535ff.).
3) Assign the various branch carbon resonances to specific branch positions and lengths using tabulated and calculated values (Lindeman, L. P., Journal of Qualitative Analytical Chemistry 43, 1971 1245ff; Netzel, D. A., et. al., Fuel, 60, 1981, 307ff). Examples TABLE-US-00002 Branch N M R Chemical Shift (ppm) 2-methyl 22.7 3-methyl 19.3 or 11.4 4-methyl 14.3 4 + methyl 19.8 Internal ethyl 10.8 Internal propyl 14.5 or 20.5 Adjacent methyls 16.5
4) Estimate relative branching density at different carbon positions by comparing the integrated intensity of the specific carbon of the methyl/alkyl group to the intensity of a single carbon (which is equal to total integral/number of carbons per molecule in the mixture). For the unique case of the 2-methyl branch, where both the terminal and the branch methyl occur at the same resonance position, the intensity was divided by two before estimating the branching density. If the 4-methyl branch fraction is calculated and tabulated, its contribution to the 4+ methyls must be subtracted to avoid double counting.
5) Calculate the average carbon number. The average carbon number may be determined with sufficient accuracy for lubricant materials by dividing the molecular weight of the sample by 14 (the formula weight of CH₂).
6) The number of branches per molecule is the sum of the branches found in step 4.
7) The number of alkyl branches per 100 carbon atoms is calculated from the number of branches per molecule (step 6) times 100/average carbon number.
8) Estimate Branching Index (B1). The B1 is estimated by ¹H N MR Analysis and presented as percentage of methyl hydrogen (chemical shift range 0.6-1.05 ppm) among total hydrogen as estimated by N M R in the liquid hydrocarbon composition.
9) Estimate Branching proximity (BP). The BP is estimated by ¹³C N M R and presented as percentage of recurring methylene carbons which are four or more carbons away from the end group or a branch (represented by a N M R signal at 29.9 ppm) among total carbons as estimated by NMR in the liquid hydrocarbon composition.
10) Calculate the Free Carbon Index (FCI). The FCI is expressed in units of carbons. Counting the terminal methyl or branch carbon as "one" the carbons in the FCI are the fifth or greater carbons from either a straight chain terminal methyl or from a branch methine carbon. These carbons appear between 29.9 ppm and 29.6 ppm in the carbon-13 spectrum. They are measured as follows:
   a. calculate the average carbon number of the molecules in the sample as in step 5,
   b. divide the total carbon-13 integral area (chart divisions or area counts) by the average carbon number from step a. to obtain the integral area per carbon in the sample,
   c. measure the area between 29.9 ppm and 29.6 ppm in the sample, and
   d. divide by the integral area per carbon from step b. to obtain FCI (EP1062306A1).

Measurements can be performed using any Fourier Transform NMR spectrometer. Preferably, the measurements are performed using a spectrometer having a magnet of 7.0 T or greater. In order to minimize non-uniform intensity data, the broadband proton inverse-gated decoupling can be used during a 6 second delay prior to the excitation pulse and on during acquisition. Samples can also be doped with 0.03 to 0.05 M Cr(acac)₃ (tris(acetylacetonato)-chromium(III)) as a relaxation agent to ensure full intensities are observed. Total experiment times can range from 4 to 8 hours.

The ¹H NMR analysis can also be carried out using a spectrometer having a magnet of 7.0 T or greater. Free induction decay of 64 co-averaged transients can be acquired, employing a 90 degree excitation pulse, a relaxation decay of 4 seconds, and acquisition time of 1.2 seconds.

The DEPT and APT sequences can be carried out according to literature descriptions with minor deviations described in the Varian or Bruker operating manuals. DEPT is Distortionless Enhancement by Polarization Transfer. The DEPT 45 sequence gives a signal all carbons bonded to protons. DEPT 90 shows CH carbons only. DEPT 135 shows CH and CH₃ up and CH₂ 180. degrees out of phase (down). APT is Attached Proton Test. It allows all carbons to be seen, but if CH and CH₃ are up, then quaternaries and CH₂ are down. The sequences are useful in that every branch methyl should have a corresponding CH. And the methyl groups are clearly identified by chemical shift and phase. Both are described in the references cited.

The branching properties of each sample can be determined by ¹³C NMR using the assumption in the calculations that the entire sample are iso-paraffinic. The naphthenes content may be measured using Field Ionization Mass Spectroscopy (FIMS).

### Branching Density

### NMR analysis.

In one embodiment, the weight percent of all molecules with at least one aromatic function in the purified mono-aromatic standard can be confirmed via long-duration carbon 13 NMR analysis. The NMR results can be translated from % aromatic carbon to % aromatic molecules (to be consistent with HPLC-UV and D 2007) knowing that 95-99% of the aromatics in highly saturated fuels are single-ring aromatics. In another test to accurately measure low levels of all molecules with at least one aromatic function by NMR, the standard D 5292-99 (Reapproved 2004) method can be modified to give a minimum carbon sensitivity of 500:1 (by ASTM standard practice E 386) with a 15-hour duration run on a 400-500 MHz NMR with a 10- 12 mm Nalorac probe. Acorn PC integration software can be used to define the shape of the baseline and consistently integrate.

Extent of branching refers to the number of alkyl branches in hydrocarbons. Branching and branching position can be determined using carbon-13 (¹³C) NMR according to the following nine-step process:
1) Identify the CH branch centers and the CH₃ branch termination points using the DEPT Pulse sequence (Doddrell, D. T.; D. T. Pegg; M. R. Bendall, Journal of Magnetic Resonance 1982, 48, 323ff.).
2) Verify the absence of carbons initiating multiple branches (quaternary carbons) using the APT pulse sequence (Patt, S. L; J . N . Shoolery, Journal of Magnetic Resonance 1982, 46, 535ff.).
3) Assign the various branch carbon resonances to specific branch positions and lengths using tabulated and calculated values known in the art (Lindeman, L. P., Journal of Qualitative Analytical Chemistry 43, 1971 1245ff, Netzel, D. A., et. al., Fuel, 60, 1981, 307ff).
4) Estimate relative branching density at different carbon positions by comparing the integrated intensity of the specific carbon of the methyl/alkyl group to the intensity of a single carbon (which is equal to total integral/number of carbons per molecule in the mixture). For the 2-methyl branch, where both the terminal and the branch methyl occur at the same resonance position, the intensity is divided by two before estimating the branching density. If the 4-methyl branch fraction is calculated and tabulated, its contribution to the 4+ methyls is subtracted to avoid double counting.
5) Calculate the average carbon number. The average carbon number is determined by dividing the molecular weight of the sample by 14 (the formula weight of CH.₂).
6) The number of branches per molecule is the sum of the branches found in step 4.
7) The number of alkyl branches per 100 carbon atoms is calculated from the number of branches per molecule (step 6) times 100/average carbon number.
8) Estimate Branching Index (B1) by ¹H N M R Analysis, which is presented as percentage of methyl hydrogen (chemical shift range 0.6-1.05 ppm) among total hydrogen as estimated by N M R in the liquid hydrocarbon composition.
9) Estimate Branching proximity (BP) by ¹³C N M R, which is presented as percentage of recurring methylene carbons-which are four or more carbons away from the end group or a branch (represented by a N M R signal at 29.9 ppm) among total carbons as estimated by NMR in the liquid hydrocarbon composition. The measurements can be performed using any Fourier Transform N M R spectrometer, e.g., one having a magnet of 7.0 T or greater. After verification by Mass Spectrometry, UV or an N MR survey that aromatic carbons are absent, the spectral width for the ¹³C N M R studies can be limited to the saturated carbon region, 0-80 ppm vs. TMS (tetramethylsilane). Solutions of 25-50 wt. % in chloroform-dl are excited by 30 degrees pulses followed by a 1.3 seconds (sec.) acquisition time. In order to minimize non-uniform intensity data, the broadband proton inverse-gated decoupling is used during a 6 sec. delay prior to the excitation pulse and on during acquisition.

Samples are doped with 0.03 to 0.05 M Cr(acac)₃ (tris(acetylacetonato)-chromium (III)) as a relaxation agent to ensure full intensities are observed. The DEPT and APT sequences can be carried out according to literature descriptions with minor deviations described in the Varian or Bruker operating manuals. DEPT is Distortionless Enhancement by Polarization Transfer. The DEPT 45 sequence gives a signal all carbons bonded to protons. DEPT 90 shows CH carbons only. DEPT 135 shows CH and CH₃ up and CH₂ 180 degrees out of phase (down). APT is attached proton test, known in the art. It allows all carbons to be seen, but if CH and CH₃ are up, then quaternaries and CH₂ are down. The branching properties of the sample can be determined by ¹³C NMR using the assumption in the calculations that the entire sample was iso-paraffinic. The unsaturates content may be measured using Field Ionization Mass Spectroscopy (FIMS).

### Branching Index

A branching index means a numerical index for measuring the average number of side chains attached to a main chain of a compound. For example, a compound that has a branching index of two means a compound having a straight chain main chain with an average of approximately two side chains attached thereto. The branching index of a product may be determined as follows. The total number of carbon atoms per molecule is determined. A preferred method for making this determination is to estimate the total number of carbon atoms from the molecular weight. A preferred method for determining the molecular weight is Vapor Pressure Osmometry following ASTM-2503, provided that the vapor pressure of the sample inside the Osmometer at 45. degree. C. is less than the vapor pressure of toluene. For samples with vapor pressures greater than toluene, the molecular weight is preferably measured by benzene freezing point depression. Commercial instruments to measure molecular weight by freezing point depression are manufactured by Knauer. ASTM D2889 may be used to determine vapor pressure. Alternatively, molecular weight may be determined from a ASTM D-2887 or ASTM D-86 distillation by correlations which compare the boiling points of known n-paraffin standards.

The fraction of carbon atoms contributing to each branching type is based on the methyl resonances in the carbon N M R spectrum and uses a determination or estimation of the number of carbons per molecule. The area counts per carbon is determined by dividing the total carbon area by the number of carbons per molecule. Defining the area counts per carbon as "A", the contribution for the individual branching types is as follows, where each of the areas is divided by area A:
2- branches=half the area of methyls at 22.5 ppm/A
3- branches=either the area of 19.1 ppm or the area at 11.4 ppm (but not both)/A
4- branches=area of double peaks near 14.0 ppm/A
4+ branches=area of 19.6 ppm/A minus the 4-branches
   internal ethyl branches=area of 10.8 ppm/A

The total branches per molecule (i.e. the branching index) is the sum of areas above. For this determination, the N M R spectrum is acquired under the following quantitative conditions: 45 degree pulse every 10.8 seconds, decoupler gated on during 0.8 sec acquisition. A decoupler duty cycle of 7.4% has been found to be low enough to keep unequal Overhauser effects from making a difference in resonance intensity.

The fuel streams produced in the hydroisomerization or Isomerization steps may be further hydrofined to remove trace oxygenates, S and N bearing molecules and various unsaturated components. Suitable hydrofinishing catalysts include noble metals from Group VINA (according to the 1975 rules of the International Union of Pure and Applied Chemistry), such as platinum or palladium on an alumina or siliceous matrix, and unsulfided Group VIIIA and Group VIB, such as nickel-molybdenum or nickel-tin on an alumina or siliceous matrix. U.S. Pat. No. 3,852,207 describes a suitable noble metal catalyst and mild conditions. Other suitable catalysts are described, for example, in U.S. Pat. No. 4,157,294, and U.S. Pat. No. 3,904,513. The non-noble metal (such as nickel-molybdenum and/or tungsten, and at least about 0.5, and generally about 1 to about 15 weight percent of nickel and/or cobalt determined as the corresponding oxides. The noble metal (such as platinum) catalyst contains in excess of 0.01 percent metal, preferably between 0.1 and 1.0 percent metal. Combinations of noble metals may also be used, such as mixtures of platinum and palladium.

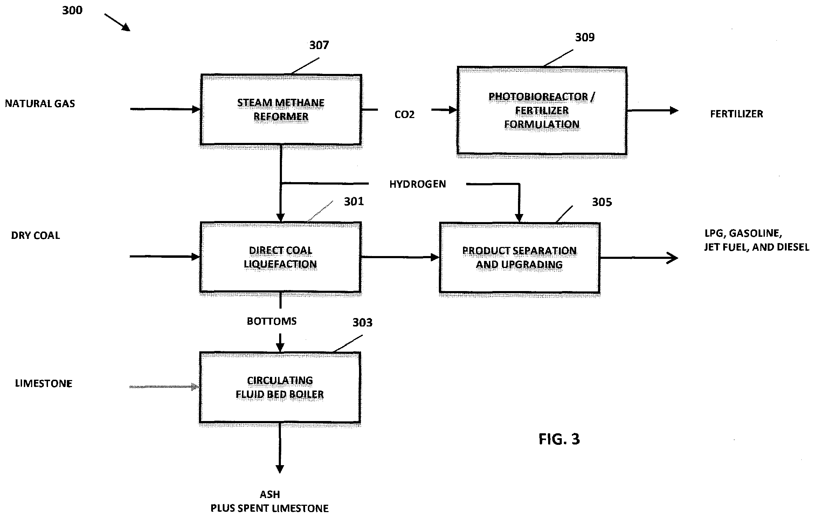

Referring now to Fig 3 of the drawings, there is illustrated an embodiment of a direct coal liquefaction and blue-green algae-based fertilizer production method and system 300 having a particularly high energy efficiency and low GHG footprint. In this embodiment in the system coal is liquefied in the DCL reactor system 301 that can be of the same design as described above, and the bottoms generated thereby are fed to a circulating fluid bed (CFB) boiler 303 that can form part of an electrical power generation system. The CFB combustion process has the advantage of having inherent pollution control. Limestone fed to the CFB boiler 303 captures SOx and removes it at the point where it is formed as the fuel burns. The relatively low combustion temperature minimizes NOx formation. By injecting ammonia from the product upgrading system 305 into the CFB boiler 303, NOx can be further reduced by half.

The liquids produced by the DCL reactor system 301 are fed to the product separation and upgrading system 305 for producing LPG, gasoline, jet fuel and/or diesel. Steam methane reformer (SM R) 307 converts natural gas to hydrogen for supply to the DCL reactor system 301 and the product upgrading system 305. The SM R 307 is the most efficient and widely applied industrial method for producing hydrogen. The process employs catalytic conversion of hydrocarbons and steam, at 1,500°F and 200 to 300 psig, to hydrogen and carbon oxides, followed by the water-gas shift reaction to convert carbon monoxide to hydrogen.

The CO₂ produced by the SM R 307 can be readily separated from the hydrogen produced using commercial technologies such as monoethanolamine (MEA). Commercial technology is available from a number of vendors including Haldor Topsoe and CB&I. The CO₂ is supplied to the photobioreactor algae and formulated biofertilizer production system 309, which preferably includes one or more closed PBRs such as described in the references identified above. The algal biomass and photosynthetic microorganisms produced by the system 309 preferably is used to produce a biofertilizer having the CO₂ terrestrial sequestration and nitrogen fixing advantages described above.

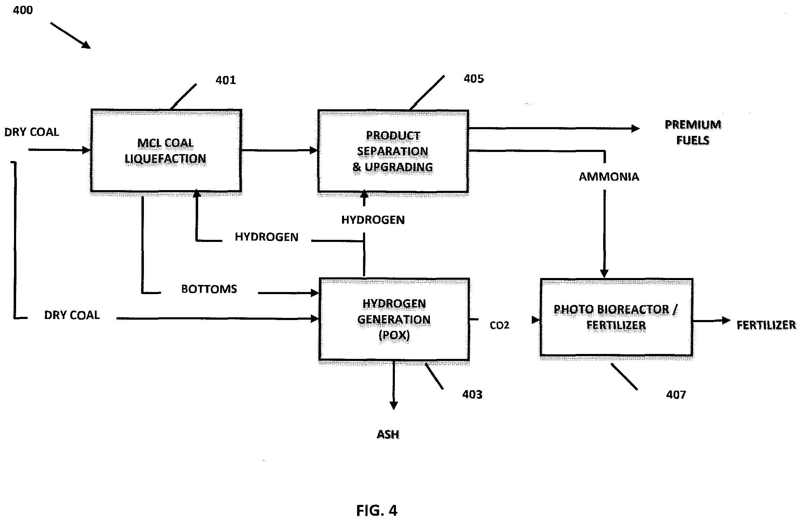

Referring now to Fig. 4 of the drawings, there is illustrated an embodiment 400 that produces a maximal amount of fertilizer so as to have an extremely small and even negative carbon footprint. In addition to producing fuels as in the embodiments described above, the embodiment of Fig. 4 can provide a large amount of carbon credits on a lifecycle basis for electric power generating plants. Coal is fed to the DCL reactor system 401 and to the POX system 403. The coal fed to the DCL reactor system 401 is liquefied in the manner described above and the products thereof are fed to the product separation and upgrading system 405 to generate premium fuels such as gasoline, diesel and jet fuel and/or chemical feedstocks. Bottoms from the DCL reactor system 401 are fed to the POX 403, in which they are gasified to generate hydrogen for supply to the DCL reactor system 401 and the product separation and upgrading system 405. The POX system 403 also generates large amounts of concentrated, pure CO₂ which is supplied to the photobioreactor algae and formulated biofertilizer production system 407 that preferably includes one or more closed PBR's such as described in the references identified above. Ammonia from the product separating and upgrading system 405 is also supplied to the photobioreactor algae and formulated biofertilizer production system 407 as a nutrient. The system 407 preferably produces algal biomass and photosynthetic microorganisms for use in a biofertilizer having the CO₂ terrestrial sequestration and nitrogen fixation advantages described above.

In the POX process, coal is partially combusted, non-catalytically, in a gasifier with oxygen typically at 2,600°F and 1,000 psig. At these conditions, the ash is converted to a liquid and flows down the inside wall of the gasifier and is collected at the bottom of the gasifier. The syngas that leaves the top of the gasifier is scrubbed of particulate matter and carbon monoxide present in the syngas can be converted to hydrogen via the water-gas shift reaction. Sulfur and CO₂ are removed in a double-stage Selexol Unit. A number of commercial vendors including Shell, Siemens, and General Electric have applied POX commercially and offer the technology for license. UOP and others license the Selexol Process.

### Biofertilizer

In the production of a preferred biofertilizer, a PBR is inoculated with a biological culture that can be drawn from its normal residence in the top centimeter of healthy undisturbed soil found in un-shaded areas having similar soil and environmental characteristics as the soil to which the biofertilizer is to be applied, or with a biological culture that includes one or more cyanobacteria strains and preferably other photosynthetic microorganisms suitable for use as a fertilizer in the location where the biofertilizer is to be used. In nature, these soil microorganisms form a biological soil crust ("BSC") that serves many functions, including gluing the soil grains in place, thereby limiting wind and water erosion, as well as providing fertilization and plant vitality. Cyanobacteria and "cyanolichens" are a primary source of fixed atmospheric nitrogen in arid ecosystems. Studies, in the western United States, have observed that between 5 to 49 cyanobacterial taxa depending on the study site. *Nostoc, Schizothrix, Anabaena,* and *Tolypothrix* are the most frequently encountered heterocystous genera. *Microcoleus* and *Phormidium* are commonly encountered non-heterocystous genera. In western Colorado, for example, *Scytonema,* a heterocystous genus, is frequently observed. Heterocysts are differentiated specialized cells responsible for nitrogen fixation. Heterocysts lack the water-splitting 0₂-evolving Photosystem II apparatus. This adaptation has evolved to eliminate the inhibition of nitrogenase activity by O₂, but still generates ATP energy by retaining photosystem-1 activity.

Many non-heterocystous cyanobacterial genera are known to contain nitrogenase and may fix nitrogen in the dark under microaerophillic or anaerobic conditions. *Microcoleus vaginatus* is an extremely important microbiotic crust component based on its frequency of occurrence and morphology. The mucilaginous encased filaments of *Microcoleus vaginatus* are highly effective in binding sand particles, thus reducing erosion and producing a stable substrate for the colonization of cyanolichens and other microorganisms. Although *Microcoleus vaginatus* may not fix nitrogen directly, it is thought that its mucilaginous sheath provides an anaerobic micro-environment and carbon source for epiphytic diazotrophic bacteria.

Cyanolichens are also a major contributor of fixed-nitrogen and microbiotic crust ground cover in desert ecosystems. Lichens are a mutualistic symbiosis between a fungus (mycobiont) and an alga (phycobiont). In most cases, the lichen phycobiont is a green alga, usually *Trebouxia,* but the cyanolichen phycobiont consists of cyanobacteria, most commonly *Nostoc, Scytonema,* or *Anabaena.* These cyanolichens are characteristically black, gelatinous in texture, and non-stratified. Certain stratified lichens inhabiting subalpine biomes, such as *Peltigera* and *Lobaria*, contain both the green *Trebouxia*, and the nitrogen-fixing cyanobacterium, *Nostoc.* For example, the cyanolichens of the arid western United States can occupy from 40 to 100% of the ground cover and make significant contributions towards soil stabilization and N₂-fixation. Depending on the soil and abiotic environment, up to 159 lichen species representing 53 genera have been observed. Some of the most commonly encountered genera include, *Collema, Placinthium, Leptogium,* and *Heppia.*

The cyanobacterial genera to be exploited may be obtained from biological soil crusts and include, but are not limited to, the following genera: *Nostoc, Anabaena, Scytonema, Tolypothrix, Calothrix, Microcoleus, Rivularia, Phormidium, Symploca, Schizothrix, Stigonema, Plectonema,* and *Chroococcus.* In addition to these cyanobacteria, it can be desirable to include eukaryotic algae such as *Chlamydomonas, Trebouxia, Scenedesmus,* for instance. In many cases, it will be desirable to include free-living nitrogen-fixing bacteria, such as *Azotobacter, Rhodospirillium,* or *Rhodopseudomonas,* for example. Other important soil bacteria such *Arthrobacter* and various *actinomycetes* including the genera, *Frankia, Nocardia, Streptomyces,* and *Micromonospora* may be included to enhance nutrient cycling. Finally, it may also be desirable to include lichenizing, saprophytic, and mycorrhizal fungi to complete the microbial complement of the basic photosynthetic biofertilizer. These heterotrophic microorganisms will be produced using standard methods.

The biofertilizer is preferably designed, in addition to providing soil nitrogen and carbon, to behave as an erosion control agent. In most cases, the biofertilizer alone will achieve the desired results. Based on the flexibility of the biofertilizer, it can be used in conjunction with traditional erosion control methods such as fibrous mulches and tackifiers thus enhancing the efficacy of these traditional products. For instance, hard-rock mine tailings, waste and overburden characteristically become acidic (pH<3) through the oxidation of sulfur by bacteria. These acidic environments inhibit seed germination and exceed the lower pH limit of cyanobacteria (pH<5). However, it has been shown that when a layer of mulch is applied to the surface, it serves as a chemical insulator that permits seed germination and the growth of the biofertilizer. The plant roots penetrate into the nitrogen-deficient acidic mine tailings and continue to grow when nitrogen is supplied by the biofertilizer.

It has it has been found that rhizobacteria are a key component of the microorganisms found in soils. It is believed that cyanobacteria particularly when present in combination with rhizobacteria act as a phytostimulator and generate organic acids including gibberellic acid and acetic acid and other mono and poly carboxylic acids, which are important stimulants for plant growth. It has further been found that different kinds of soil formation have different complements of naturally occurring microorganisms that contribute to to the fertility of the soil for various crop and natural plant species to take root and flourish. For example, the Desert Institute of the Chinese Academy of Sciences has found in desert soils that, in sand, the primary surface layer microorganisms were found to be *Fragilaria, Oscillatoria willei*, and *Phormidium okenii.* Where the surface layer is an algal crust the primary microorganisms were found to be *Synechococcus parvus, Tychonema granulatum* and *Phormidium retzli.* Where the surface layer is a lichen crust the primary microorganisms were found to be *Oscillatoria wille, Oscillatoria carboniciphila* and *Phormidium retzli.* In the case of the moss crust surface layer, the primary microorganisms were found to be *Synechococcus parvus*, *Synechocystis pavalekii* and *Phormidium retzli.* It is particularly beneficial to nurture such natural colonies to form, particularly in arid regions were reestablishment of natural flora can be beneficial to soil stabilization and to the increased production of natural plant colonies in replenishing the soil with carbon and other nutrients. The Institute has reported that certain species of these microorganisms are prevalent in soil samples in the Gobi and nearby deserts in China, and these species are of particular interest as potential members of the population of organisms to be incorporated into the final biofertilizer formulation. For example, see the recent report by Yanmei Liu et al on "The Effects of Soil Crusts on Soil Nematode Communities Following Dune Stabilization in the Tennger Desert, Northern China" Applied Soil Ecology, vol 49, pp 118-124 (2011).

Many of the microorganisms in the BSC are also photosynthetic and draw their energy from sunlight such that they can, in-tum, manufacture and provide nutrition and fixed nitrogen to cohort microorganisms that are not photosynthetic or are found deeper in the soil. The actions of the BSC, and the deeper cohort microorganisms it supplies nutrition to, work together to stabilize soil and draw plant available nutrition from the grains of soil into the soil matrix over time. In addition, the dominant cyanobacteria component of BSC fixes carbon as well as nitrogen from the atmosphere. Beginning with BSC, the combined actions of these microorganisms create conditions benefiting the establishment and growth of vascular plants like grasses, shrubs and crops. In effect, the BSC is a naturally occurring solar powered fertilizer that lives on the surface of bare earth making it suitable and beneficial for the establishment of vascular plants over time. However, because BSC microorganisms reproduce slowly in dry climates and are not very motile, physical disturbances like tilling, livestock grazing, and fire can halt the BSC's beneficial effects for the soil and the BSC, and these benefits can take decades or centuries in dry climates to naturally restore. The production of the preferred biofertilizer rapidly reproduces naturally occurring BSC microorganisms at an industrial scale in a PBR. The microorganisms are then carefully compounded to form "inoculant seeds" of these microorganisms that constitute the preferred biofertilizer, and that are spread onto land presently lacking healthy soil crust colonies, thus accelerating the natural recovery of the soil. As the biofertilizer propagates on the soil surface, it draws down increasing amounts of carbon from atmospheric CO₂ into the soil where that carbon becomes part of a living sustainable microbiological community and effectively sequesters this atmospheric carbon into the soil. Through soil inoculation with the preferred biofertilizer, its natural propagation on the soil and secondary vascular plant growth enhancement, it has been estimated that the conversion of 1 ton of CO₂ into the preferred biofertilizer, which is then applied onto suitable soils, can cause the drawdown of up to 50 tons of CO₂ from the atmosphere annually through direct photosynthetic uptake of atmospheric gasses by that soil.

The cyanobacteria and their soil consortia used to produce the biofertilizer are preferably cultured into an inoculum in a manner taught by U.S. Patent Application Publication No. US 2008/0236227 to Flynn, the contents of which are hereby incorporated by reference in their entirety, (herein after referred to as "Flynn") and used to inoculate an amplifying PBR, also taught by Flynn, where the culture can be rapidly grown in liquid media via ready access to nutrients, carbon dioxide, sunlight and hydraulic mixing. The PBR may be fed by sunlight, nutrients and a carbon source that is most commonly carbon dioxide, but that may be a fixed form such as sodium bicarbonate or other bio-available forms.

A preferred method for producing the biofertilizer includes the following steps:
(1) Isolating the important photosynthetic biological soil crust microorganisms to produce a polyspecies culture that closely reflects the native microbial species composition;
(2) Cultivating the culture in a PBR, preferably under controlled conditions designed to maximize biomass productivity;
(3) Harvesting the produced biomass by, for example, a simple gravity-driven sedimentation and filtration, clarification, or centrifugation;
(4) Preserving the biomass by, e.g., using refractance window drying technology, or other methods such as air drying, spray drying, vacuum drying, or freezing such that the cells remain viable;
(5) Pulverize, flake, or powder the dried cyanobacteria to facilitate packaging, storage, shipment, and final dissemination of the biofertilizer. After growing in the PBR, the soil microorganisms being harvested and compounded using admixes and coatings to create the product biofertilizer, the biofertilizer can be spread upon farmlands or damaged land using standard agricultural practices, such as crop dusting, mixing with irrigation water or applying with spreading machines. Once on the soil surface, the natural availability of carbon dioxide and nitrogen in air, along with available participation or irrigation water and sunlight, causes the biofertilizer to induct a growing colony of soil microorganisms in proportion with the suitability of growth conditions for that specific consortium of microorganisms. The consortium of microbes in a locally adapted biofertilizer is preferably picked from local soil samples representing the best target outcome that could be expected from a soil crust reseeding effort of similar local soils. When this is done and the biofertilizer is spread to sufficient surface density, then the crust will reestablish at an accelerated rate well in advance of natural propagation. In land reclamation efforts, sufficient application density is approximately 0.1 to 2 biofertilizer particles per square cm. In agricultural applications where accelerated fertilization performance is required, sufficient application density is approximately 1 to 20 biofertilizer particles per square cm.

As microorganisms grow and propagate in and on the soil, their uptake of CO₂ from the atmosphere increases proportionate with the population size, impinging sunlight, water availability, soil type and the occurrence of secondary vascular plant growth that might further increase the net primary productivity of the soil. The amount of CO₂ drawn down from the atmosphere will vary widely dependant on these factors. It is estimated that if a crust is allowed to grow to maturity in a land reclamation application, that it will draw down from the atmosphere approximately 100 grams of CO₂ per square meter per year.

The soil sample is preferably drawn from a desired target outcome soil patch that represents the best and most desired microbiological outcome for the treated soil, and that is similar in non-biological constitution and environmental factors to the soil in the area to be treated. In this way, a consortium of microorganisms can be specifically selected to manufacture a particular regional type of biofertilizer that includes microorganisms most favored to survive, thrive and fertilize on the targeted soil to be treated in that region.

The purpose of the inoculation PBR is to obtain the organisms from the target outcome soil and begin growing a population facsimile within the PBR's liquid medium. The population generated by the inoculation PBR should have substantially the same or otherwise sufficient microorganism consortia members and in roughly substantially the same or otherwise sufficient balance as they were present natively in the soil. The PBR operator uses input and output population and growth media assay data to adjust growth input parameters such as light, pH, temperature, CO2 and nutrient levels, as well as mixing speed to effect the desired growth rate and population balance characteristics on the output of the incubator. In a similar fashion, the amplifier and production PBR operator looks at the population and growth media assay between the input and output of the PBRs and adjusts the same growth conditions to effect the desired result. In some cases, the desired product population ratio may be different from that found in the target outcome soil, but will affect a better result upon application via that difference.

The pH and rate of photosynthesis in the PBR system can be measured using the PT4 Monitor, available from Point Four Systems Inc. (Richmond, British Columbia Canada), which includes the controller, acquisition software, dissolved oxygen, pH, and temperature probes. The difference in dissolved oxygen between the lower and upper probe arrays provides a measure of photosynthesis. Likewise, the difference in pH between the lower and upper probe arrays is a measure of CO₂ consumption. Under illumination, the microorganisms will photosynthesize and assimilate CO₂ causing the pH of the medium to rise. When the pH increases to a chosen set point, preferably pH 7.5, the controller will introduce 100% CO₂ into the PBR, which will cause the pH to drop as a result of the formation of carbonic acid and related complexes.

The output of the PBR may be fed into filtering and drying belts in which various optional admixes can be applied. The resultant dry flake and its optional coating may then be granulated to become the biofertilizer. The final biofertilizer product can be distributed and applied to soil via various agricultural and land restoration spreaders. Advantageously, the biofertilizer pellets can be broadcast by a spinning spreader or aircraft such that they are not blown away by the ambient wind. The biofertilizer can also be mixed with irrigation water and sprayed on crops. The various admixes optionally to be included also desirably remain physically associated with the microorganism consortium in the same relative proportions, even as the composite admix/biomass flake is reduced in size by granulation. By even layering and infusing of the admix homogeneously across the flake as the flake is being generated, then these relative proportions of admix/biomass can be maintained during the granulation and particle coating process. The dry admix components may be further added as the biomass mat begins to consolidate, which helps to mechanically consolidate them with the biomass by entrapping some of the dry admix in the filaments of the consolidating cyanobacteria. The wet admix is typically, but not exclusively, a sugar-based composition of xero-protectants and heterotrophic consortium member nutrition additive that serve to bind and glue all the components together as it dries. Using an actual mucilage or other watersoluble glue for this purpose, or a solvent based but UV degradable binder, can also be considered for this purpose.

The following are optional admixes and their purpose:
1) Anti-oxidants such as beta carotene can preserve the biofertilizer during the drying process and in storage.
2) Xero-protectants such as sucrose and other sugars, or a biologically derived xeroprotectant called trehalose can prevent cell damage from rapid desiccation and extended desiccation over time.
3) Growth nutrients include micro nutrients needed by all soil microorganisms as taught by Flynn including sugars to feed the non-photosynthetic cohorts during the initial stages of establishment.
4) Sand or clay fillers serve two purposes. One is to increase the weight density of the resultant granulated particles thereby making them more aerodynamically spreadable from aircraft and land-based spreaders and resistant to wind currents. The other purpose is to provide a non-damaging location for fracture lines between the desiccated microorganisms during granulation that does not split through the microorganism itself.
5) Spread pattern tracers may be fluorescent additives. Another tracing tag may be the use of inheritable but non-operational unique gene sequences within one of the microorganisms that will propagate at the same rate and with the same spatial characteristics as the biofertilizer propagates. This will allow a researcher or carbon credit auditor to visit a patch of soil months or years after initial application of the biofertilizer and know how much of the soil crust or under-earth biomass is directly due to the propagation and beneficial actions of the specifically tagged biofertilizer.
6) Vascular plant seeds like restorative grasses or actual crop seeds may become part of admix. In this case the biofertilizer would be designed to work in biological concert with the embedded vascular plant seeds to achieve and maximize the desired restorative of fertilizing result.
7) A tackifier may be added to the admix in order to quickly bind the particle with other soil grains upon first environmental wetting to prevent further shifting by wind or water erosion.
8) Other microorganisms may be added to either the dry mix or to the wet mix. These other microorganisms may be chosen for their auxiliary properties like being a good tackifier or they may be chosen because they are an important part of the biological consortium of the biofertilizer; yet for various reasons such as growth media type incompatibility or susceptibility to predation they were not able to be co-grown in the same PBR as the rest of the biofertilizer consortium members.

Biologies may also be spray coated onto the exterior of the particle. In this context "biologies" can refer to whole living or dead cells or bio-active substances that affect the receptivity of the soil to being colonized by the biofertilizer microorganisms. Alternatively, these substances may be intended to prevent the consumption or destruction of the biofertilizer by other living organisms such as insects, other microorganisms, birds or other living creatures.

## Claims

1. A method for converting a solid carbonaceous material to liquid fuels and cyanobacteria based biofertilizer, comprising the steps of:
(a) directly liquefying a coal containing solid carbonaceous material by subjecting said material to elevated temperatures and pressures in the presence of a solvent and a molybdenum containing catalyst for a time sufficient for producing hydrocarbon liquids and byproduct CO₂;
(b) upgrading hydrocarbon liquids produced by step (a) to liquid fuels and byproduct ammonia;
(c) producing hydrogen and byproduct CO₂ from a carbonaceous feed, and supplying at least a portion of said hydrogen as inputs to said direct liquefaction and said upgrading steps; and
(d) reproducing nitrogen-fixing cyanobacteria in a photobioreactor with the use of byproduct C0₂ produced by one or both of said direct liquefaction and hydrogen producing steps and ammonia produced by said upgrading step, the nitrogen fixing cyanobacteria naturally occurring in a type of soil to which the biofertilizer is to be applied; and
(e) producing a biofertilizer incorporating the cyanobacteria.

2. The method of Claim 1, wherein said molybdenum containing catalyst is produced in situ from a PMA catalyst precursor, and wherein phosphorus obtained from said catalyst precursor is supplied to said photobioreactor as a nutrient.

3. The method of claim 1 or 2, wherein said hydrogen producing step includes gasifying said carbonaceous feed in a partial oxidation reactor or a gasifier and wherein said carbonaceous feed includes bottoms from said direct liquefaction step or from said upgrading step or from both.

4. The method of any preceding claim, wherein said hydrogen producing step includes reacting natural gas in a steam methane reformer to produce hydrogen and byproduct CO₂.

5. The method of any preceding claim, further including feeding bottoms produced by said direct liquefaction step and limestone to a circulating fluid bed boiler for powering an electric power generation system.

6. The method of any preceding claim, further including extracting lipids from cyanobacteria produced in said photobioreactor and converting said extracted lipids to hydrocarbon liquids.

7. The method of claim 6, further comprising supplying the biomass residues remaining after the extraction of said lipids as an input to said hydrogen producing step.

8. The method of claim 7, further comprising supplying hydrogen produced by said hydrogen producing step to the lipid conversion step.

9. The method of any preceding claim, wherein the cyanobacteria being reproduced to produce said inoculant are obtained from biological soil crust.

10. The method of any preceding claim, wherein the biofertilizer further includes one or more additional microorganisms selected from the groups comprising free-living nitrogen-fixing heterotropic bacteria, actinomycetes, photosynthetic bacteria, mycorrhizal or lichenizing fungi, and combinations thereof, and symbiotic nitrogen-fixing microorganisms including rhizobacteria.

11. The method of any preceding claim, wherein the biofertilizer is transformed into a dormant state by a technique selected from the group consisting of spray drying, refractance-window drying, solar drying, air drying, or freeze drying.

12. The method of claim 11, wherein xeroprotectant additives including one or more of sorbitol, mannitol, sucrose, sorbitan monostereate, dimethyl sulfoxide, methanol, beta-carotene, and beta-mercaptoethanol are used to increase post drying viability.

13. The method of any preceding claim, wherein the biofertilizer is combined with an additive selected from the group comprised of fibrous, cellulosic mulch material, polymeric tackifiers, clays, geotextiles, and combinations.

## Patentansprüche

1. Verfahren zur Umwandlung eines festen kohlenstoffhaltigen Materials in flüssige Brennstoffe und Biodünger auf Cyanobakterienbasis, umfassend die Schritte:
(a) direktes Verflüssigen einer Kohle, die festes kohlenstoffhaltiges Material umfasst, indem das Material erhöhten Temperaturen und Drücken in Gegenwart eines Lösungsmittels und eines Molybdän-haltigen Katalysator ausgesetzt wird für eine Zeit, die ausreichend ist, um flüssige Kohlenwasserstoffe und das Nebenprodukt CO₂ herzustellen;
(b) Aufarbeiten von den in Schritt (a) hergestellten flüssigen Kohlenwasserstoffen zu flüssigen Brennstoffen und dem Nebenprodukt Ammoniak;
(c) Herstellen von Wasserstoff und dem Nebenprodukt CO₂ aus einer kohlenstoffhaltigen Beschickung und Zuführen mindestens eines Teils des Wasserstoffs als Eingaben zu der direkten Verflüssigung und den Aufarbeitungsschritten; und
(d) Reproduzieren von Stickstoff-fixierenden Cyanobakterien in einem Photobioreaktor unter Verwendung des Nebenprodukts CO₂, hergestellt durch einen oder beide des direkten Verflüssigungsschritts und der Wasserstoff herstellenden Schritte und Ammoniak, das durch den Aufbereitungsschritt hergestellt wird, wobei die Stickstoff-fixierenden Cyanobakterien in einem Bodentyp natürlich vorkommen, auf dem der Biodünger aufgetragen werden soll; und
(e) Herstellen eines Biodüngers, der die Cyanobakterien enthält.

2. Verfahren nach Anspruch 1, wobei der Molybdän-enthaltende Katalysator aus einem PMA Katalysatorvorläufer in situ hergestellt wird und wobei aus dem Katalysatorvorläufer erhaltener Phosphor dem Photobioreaktor als Nährstoff zugeführt wird.

3. Verfahren nach Anspruch 1 oder 2, wobei der Schritt der Wasserstoffproduktion das Vergasen der kohlenstoffhaltigen Beschickung in einem Teiloxidationsreaktor oder einem Vergaser umfasst und wobei die kohlenstoffhaltige Beschickung Böden aus dem direkten Verflüssigungsschritt oder aus dem Aufbereitungsschritt oder aus beiden umfasst.

4. Verfahren nach einem der vorhergehenden Ansprüche, bei dem der Schritt der Wasserstoffherstellung die Umsetzung von Erdgas in einem Dampf-Methan-Reformer zur Herstellung von Wasserstoff und dem Nebenprodukt CO₂ umfasst.

5. Verfahren nach einem der vorhergehenden Ansprüche, das ferner das Zuführen von durch den direkten Verflüssigungsschritt hergestelltem Boden und Kalkstein zu einem zirkulierenden Wirbelbettkessel zum Antreiben eines Stromerzeugungssystems umfasst.

6. Verfahren nach einem der vorhergehenden Ansprüche, das ferner das Extrahieren von Lipiden aus Cyanobakterien, die in dem Photobioreaktor hergestellt wurden, und Umwandeln der extrahierten Lipide in flüssige Kohlenwasserstoffe umfasst.

7. Verfahren nach Anspruch 6, das ferner das Zuführen der Biomasserückstände, die nach der Extraktion der Lipide verbleiben, als Eingabe in den Schritt der Wasserstoffproduktion umfasst.

8. Verfahren nach Anspruch 7, das ferner das Zuführen von Wasserstoff, der durch den Schritt der Wasserstoffproduktion hergestellt wurde, zu dem Schritt der Lipidumwandlung umfasst.

9. Verfahren nach einem der vorhergehenden Ansprüche, wobei die Cyanobakterien, die zur Herstellung des Impfmittels reproduziert werden, aus biologischer Bodenkruste erhalten werden.

10. Verfahren nach einem der vorhergehenden Ansprüche, wobei der Biodünger ferner einen oder mehrere zusätzliche Mikroorganismen umfasst, die aus den Gruppen ausgewählt sind, umfassend frei lebende Stickstoff-fixierende heterotrope Bakterien, Actinomyceten, photosynthetische Bakterien, Mykorrhiza- oder lichenisierende Pilze und Kombinationen davon und symbiotische Stickstoff-fixierende Mikroorganismen einschließlich Rhizobakterien.

11. Verfahren nach einem der vorhergehenden Ansprüche, wobei der Biodünger mittels einer Technik in einen Ruhezustand umgewandelt wird, die aus der Gruppe ausgewählt ist, bestehend aus Sprühtrocknen, Brechungsfenstertrocknen, Solartrocknen, Lufttrocknen oder Gefriertrocknen.

12. Verfahren nach Anspruch 11, wobei xeroprotektive Additive, umfassend eines oder mehrerer von Sorbit, Mannit, Saccharose, Sorbitanmonostereat, Dimethylsulfoxid, Methanol, Beta-Carotin und Beta-Mercaptoethanol, verwendet werden, um die Lebensfähigkeit nach dem Trocknen zu erhöhen.

13. Verfahren nach einem der vorhergehenden Ansprüche, wobei der Biodünger mit einem Additiv kombiniert wird, das aus der Gruppe ausgewählt ist, bestehend aus fasrigem cellulosischen Mulchmaterial, polymeren Klebrigmachern, Tonen, Geotextilien und Kombinationen davon.

## Revendications

1. Procédé de conversion d'une substance carbonée solide en combustibles liquides et biofertilisant à base de cyanobactéries, comprenant les étapes de :
(a) liquéfaction directe d'un charbon contenant une substance carbonée solide par soumission de ladite substance à des températures et pressions élevées en présence d'un solvant et d'un catalyseur contenant du molybdène pendant un temps suffisant pour produire des liquides hydrocarbonés et du sous-produit CO₂ ;
(b) valorisation de liquides hydrocarbonés produits par l'étape (a) en combustibles liquides et sous-produit ammoniaque ;
(c) production d'hydrogène et de sous-produit CO₂ à partir d'une alimentation carbonée, et fourniture d'au moins une portion dudit hydrogène en entrées auxdites étapes de liquéfaction directe et de valorisation ; et
(d) reproduction de cyanobactéries fixant l'azote dans un photobioréacteur par l'utilisation de sous-produit CO₂ produit par l'une desdites étapes de liquéfaction directe et de production d'hydrogène ou les deux et d'ammoniaque produite par ladite étape de valorisation, les cyanobactéries fixant l'azote apparaissant naturellement dans un type de sol auquel le biofertilisant doit être appliqué ; et
(e) production d'un biofertilisant incorporant les cyanobactéries.

2. Procédé selon la revendication 1, dans lequel le catalyseur contenant du molybdène est produit *in situ* à partir d'un précurseur de catalyseur PMA, et dans lequel du phosphore obtenu à partir dudit précurseur de catalyseur est fourni audit photobioréacteur en tant que nutriment.

3. Procédé selon la revendication 1 ou 2, dans lequel ladite étape de production d'hydrogène inclut la gazéification de ladite alimentation carbonée dans un réacteur d'oxydation partiel ou un gazéifieur et dans lequel ladite alimentation carbonée inclut des résidus issus de ladite étape de liquéfaction directe ou de ladite étape de valorisation ou des deux.

4. Procédé selon une quelconque revendication précédente, dans lequel ladite étape de production d'hydrogène inclut la mise en réaction de gaz naturel dans un reformeur de méthane à la vapeur pour produire de l'hydrogène et du sous-produit CO₂.

5. Procédé selon une quelconque revendication précédente, incluant en outre l'alimentation d'une chaudière à lit fluidisé circulant en résidus produits par ladite étape de liquéfaction directe et en calcaire afin d'alimenter un système de production d'électricité.

6. Procédé selon une quelconque revendication précédente, incluant en outre l'extraction de lipides de cyanobactéries produites dans ledit photobioréacteur et la conversion desdits lipides extraits en liquides hydrocarbonés.

7. Procédé selon la revendication 6, comprenant en outre la fourniture des résidus de biomasse restant après l'extraction desdits lipides en entrée à ladite étape de production d'hydrogène.

8. Procédé selon la revendication 7, comprenant en outre la fourniture d'hydrogène produit par ladite étape de production d'hydrogène à l'étape de conversion de lipide.

9. Procédé selon une quelconque revendication précédente, dans lequel les cyanobactéries qui sont reproduites pour produire ledit inoculant sont obtenues à partir de croûte de sol biologique.

10. Procédé selon une quelconque revendication précédente, dans lequel le biofertilisant inclut en outre un ou plusieurs microorganismes additionnels choisis dans les groupes comprenant des bactéries hétérotropes libres fixant l'azote vivant librement, des actinomycètes, des bactéries photosynthétiques, des champignons mycorhizateurs ou lichénisants, et leurs combinaisons, et des microorganismes symbiotiques fixant l'azote dont les rhizobactéries.

11. Procédé selon une quelconque revendication précédente, dans lequel le biofertilisant est transformé en un état dormant par une technique choisie dans le groupe consistant en le séchage par pulvérisation, le séchage par fenêtre de réfraction, le séchage solaire, le séchage à l'air, ou la cryodessiccation.

12. Procédé selon la revendication 11, dans lequel des additifs xéroprotecteurs incluant un ou plusieurs parmi le sorbitol, le mannitol, le saccharose, le monostéarate de sorbitan, le diméthyl sulfoxyde, le méthanol, le bêta-carotène, et le bêta-mercaptoéthanol sont utilisés pour augmenter la viabilité post-séchage.

13. Procédé selon une quelconque revendication précédente, dans lequel le biofertilisant est combiné avec un additif choisi dans le groupe composé de matière de paillis cellulosique et fibreuse, agents poisseux polymères, argiles, géotextiles, et combinaisons.
